# NOUVEAU FASCICULE DE BREVET EUROPEEN

(11) **EP 1 464 322 B2**
(45) Date de publication et mention de la décision concernant l'opposition: **01.06.2022**
(45) Mention de la délivrance du brevet: 05.06.2013
(21) Numéro de dépôt: 04290864.0
(22) Date de dépôt: 01.04.2004
(51) Int. Cl.: A61K 8/44, A61Q 5/10, A61Q 19/02, A61K 8/49

(54) **Utilisation de compositions comprenant un colorant fluorescent et un tensioactif amphotère ou non ionique particuliers pour colorer avec un effet eclaircissant des matières kératiniques humaines**
Verwendung von einen ausgewählten fluoreszierenden Farbstoff und ein amphoterisches oder nicht-ionisches Tensid enthaltenden Zusammensetzungen zum Färben der menschlichen keratinischen Fasern mit einem aufhellenden Effekt.
Use of compositions containing a chosen fluorescent dye and a chosen amphoteric or nonionic surfactant for dyeing human kerateous fibres with a bleaching effect

(30) Priorité: 01.04.2003 FR 0304034
(43) Date de publication de la demande: 06.10.2004
(73) Titulaire: L'Oréal, 75008 Paris (FR)
(72) Inventeur: Pourille-Grethen, Chrystel, 92110 Clichy (FR); Plos, Grégory, 75011 Paris (FR)
(74) Mandataire: L'Oreal

(56) Documents cités:
- EP-A1- 0 624 362
- EP-A2- 1 133 977
- EP-A2- 1 166 753
- EP-A2- 1 219 285
- EP-B1- 1 432 390
- WO-A-94/02022
- WO-A-02/074270
- WO-A-03/029359
- WO-A1-03/028685
- DE-A- 19 923 438
- GB-A- 1 554 331
- US-A1- 2002 004 956
- US-A1- 2003 055 268
- US-B2- 7 364 613
- D. WILLIAMS ET AL., ED.: "Chemistry and technology of the cosmetics and toiletries industry, ed.2, 1996" 2000, BLACKIE ACADEMIC , GB , XP002270544 * page 77 - page 78; tableau 2.22 *
- M. SCHLOSSMANN ED.: "The chemistry and manufacture of cosmetics: formulating. Vol.2 ed.3, 2000" 2000, ALLURED PUB. , USA 277870 , XP002270545 * page 522 - page 526 *
- Zviak: "Science des traitements capillaires", 1988
- Umbach, Wilfried: "Kosmetik. 2. Auflage", 2002 page 306,
- Beilstein Recherche (Reaxys) zu 1,1'-Diethyl-2,2'-Cyanin lodid (CAS-Nr. 977-96-8; 63902-24-9)
- Baruah et al: J. Phys. Chem. A, vol. 110, 2006, pages 415-425,
- Màrtire et al: "Chemical Paper", , 2014,
- Haidekker et al: Bioorganic Chemistry, vol. 33, 2005, pages 415-425,
- "Hair Preparation", Ullmann's Encyclopedia, 2006,
- Kirk-Othmer Encyclopedia of Chemical technology. Hair preparation. 1994
- Kirk-Othmer Encyclopedia of Chemical technology. Hair preparation. 2008

## Description

L'invention concerne l'utilisation d'une composition cosmétique comprenant au moins un colorant fluorescent et au moins un tensioactif amphotère et/ou non ionique particuliers pour colorer avec un effet éclaircissant les fibres kératiniques humaines pigmentées ou colorées artificiellement, de nouvelles compositions et les procédés mettant en oeuvre ces compositions, en l'absence de colorants d'oxydation et d'agents oxydants.

Dans le domaine capillaire, Il existe principalement deux grands types de coloration capillaire.
Le premier est la coloration semi-permanente, ou coloration directe, qui fait appel à des colorants capables d'apporter à la coloration naturelle des cheveux, une modification plus ou moins marquée résistant à plusieurs shampooings. Ces colorants sont appelés colorants directs et peuvent être mis en oeuvre de deux manières différentes. Les colorations peuvent être réalisées par application directe sur les fibres kératiniques de la composition contenant le ou les colorants directs ou par application d'un mélange réalisé extemporanément d'une composition contenant le ou les colorants directs avec une composition contenant un agent décolorant oxydant qui est de préférence l'eau oxygénée. On parle alors de coloration directe éclaircissante.
Le deuxième est la coloration permanente ou coloration d'oxydation. Celle-ci est réalisée avec des précurseurs de colorants dits "d'oxydation" qui sont des composés incolores ou faiblement colorés qui une fois mélangés à des produits oxydants, au moment de l'emploi, peuvent donner naissance par un processus de condensation oxydative à des composés colorés et colorants. Il est souvent nécessaire d'associer aux bases d'oxydation et coupleurs, un ou plusieurs colorants directs afin de neutraliser ou de rabattre les nuances trop en reflets rouges, orangés ou dorés, ou au contraire d'accentuer ces reflets rouges, orangés ou dorés.
Parmi les colorants directs disponibles, les colorants directs nitrés benzéniques ne sont pas suffisamment puissants, les indoamines, les colorants quinoniques ainsi que les colorants naturels présentent une faible affinité pour les fibres kératiniques et de ce fait conduisent à des colorations qui ne sont pas assez résistantes vis-à-vis des différents traitements que peuvent subir les fibres, et en particulier vis à vis des shampooings.

En outre, il existe un besoin d'obtenir un effet d'éclaircissement des fibres kératiniques humaines. Cet éclaircissement est obtenu classiquement par un procédé de décoloration des mélanines du cheveu par un système oxydant, généralement constitué par du peroxyde d'hydrogène associé ou non à des persels. Ce système de décoloration présente l'inconvénient de dégrader les fibres kératiniques et d'altérer leurs propriétés cosmétiques.

La présente invention concerne donc des compositions permettant d'apporter des solutions aux problèmes évoqués ci-dessus, c'est-à-dire présentant une bonne affinité tinctoriale pour les fibres kératiniques, des bonnes propriétés de ténacité vis-à-vis des agents extérieurs, et en particulier vis-à-vis des shampooings, et qui permettent également d'obtenir un éclaircissement sans altération de la fibre.

Il a donc été trouvé de manière inattendue et surprenante que l'utilisation de colorants fluorescents, en particulier ceux dans la gamme des orangés, lorsqu'ils étaient mis en présence de tensioactifs particuliers permettait d'atteindre ces objectifs.

Des colorants fluorescents tels que l'Acid Red 94 et l'Acid Red 51 sont connus pour détruire ou inactiver les protéines et les micro-organismes. Ces colorants fluorescents sont associés à des tensioactifs (WO 94/02022).

D'autres colorants fluorescents « bleu de méthylène », l' « Auramine COO » ou la « Safranine » ont été utilisés associés à des tensioactifs de type bétaïne pour colorer des cheveux clairs ou des cheveux en foncé « ton sur ton » (GB 986 712 et US 2 763 269). Un autre document mentionne l'utilisation de mélange de colorants directs pouvant contenir des colorants fluorescents et des tensioactifs pour colorer des cheveux gris ou blancs en foncés (JP 05 246 831).

La présente invention a donc pour premier objet l'utilisation d'une composition comprenant dans un milieu cosmétiquement acceptable, au moins un colorant fluorescent dans la gamme des orangés soluble dans le milieu choisi parmi les colorants fluorescents appartenant aux familles suivantes : les naphtalimides ; les coumarines cationiques ou non ; les xanthénodiquinolizines ; les azaxanthènes ; les naphtolactames , les azlactones ; les oxazines ; les thiazines ; les dioxazines ; les colorants fluorescents monocationiques ou polycationiques de type azoïque, azométhinique, ou méthinique et au moins un tensioactif amphotère choisi parmi les bétaïnes et les dérivés d'imidazolium et/ou au moins un tensioactif non ionique choisi parmi les alkylpyrrolidones, les éthers d'alcools gras oxyalkylénés ou glycérolés, les esters d'acides gras de monoalcools oxyalkylénés ou glycérolés, pour colorer avec un effet éclaircissant les fibres kératiniques humaines pigmentées ou colorées artificiellement, en l'absence de colorants d'oxydation et d'agents oxydants.

Un second objet de l'invention concerne une composition telle que revendiquée à la revendication 20.

Un troisième objet de l'invention concerne un procédé pour colorer avec un effet éclaircissant les fibres kératiniques humaines pigmentées ou colorées artificiellement tel que revendiqué à la revendication 36.

Les compositions utilisées selon l'invention permettent en particulier une meilleure diffusion du colorant fluorescent dans les fibres kératiniques humaines pigmentées ou colorées artificiellement, ce qui se traduit par un effet de fluorescence accru, par un effet d'éclaircissement supérieur et donc aussi par une luminosité supérieure à ce qui est obtenu avec le colorant fluorescent utilisé seul. On constate également une meilleure ténacité du résultat vis-à-vis des lavages ou shampooings.

Mais d'autres caractéristiques et avantages de la présente invention apparaîtront plus clairement à la lecture de la description et des exemples qui vont suivre.

A moins d'une indication différente, les bornes des gammes de valeurs qui sont données dans la description, sont incluses dans ces gammes.

Ainsi que cela a été indiqué auparavant, la présente invention a pour objet l'utilisation d'une composition comprenant dans un milieu cosmétiquement acceptable, au moins un colorant fluorescent dans la gamme des orangés soluble dans le milieu choisi parmi les colorants fluorescents appartenant aux famille suivantes : les naphtalimides ; les coumarines cationiques ou non ; les xanthénodiquinolizines ; les azaxanthènes ; les naphtolactames , les azlactones ; les oxazines ; les thiazines ; les dioxazines ; les colorants fluorescents monocationiques ou polycationiques de type azoïque, azométhinique, ou méthinique et au moins un tensioactif amphotère choisi parmi les bétaïnes et les dérivés d'imidazolium et/ou au moins un tensioactif non ionique choisi parmi les alkylpyrrolidones, les éthers d'alcools gras oxyalkylénés ou glycérolés, les esters d'acides gras de monoalcools oxyalkylénés ou glycérolés, pour colorer avec un effet éclaircissant les fibres kératiniques humaines pigmentées ou colorées artificiellement, en l'absence de colorants d'oxydation et d'agents oxydants.

Les tensioactifs amphotères de type bétaïne convenables à la présente invention sont de préférence choisis parmi les alkyl(C₈-C₂₀)bétaïnes, les alkyl(C₈-C₂₀)amidoalkyl (C₁-C₈)bétaïnes, les alkyl(C₈-C₂₀)amidoalkyl(C₁-C₈)sulfobétaines, et les sulfobétaïnes. De préférence, les tensioactifs amphotères de type bétaïne convenables à la présente invention sont de préférence choisis parmi les alkyl(C₈-C₂₀)bétaïnes, les alkyl(C₈-C₂₀)amidoalkyl(C₁-C₈)sulfobétaines, et les sulfobétaïnes.

A titre d'exemples on peut citer tout particulièrement les composés classés dans le dictionnaire CTFA, 9ème édition, 2002, sous les dénominations Coco-betaïne, Lauryl-betaïne, Cetyl-betaïne, Coco/oleamidopropyl-betaïne Cocamidopropyl-betaïne, Palmitamidopropyl-betaïne, Stearamidopropyl-betaïne Cocamidoethyl-betaïne, Cocamido propyl-hydroxy-sultaïne, Oleamidopropyl-hydroxysultaïne Coco-hydroxy-sultaïne, Lauryl-hydroxy-sultaïne, Coco-sultaïne, seuls ou en mélanges.

En ce qui concerne les dérivés d'imidazolium, ces derniers sont avantageusement choisis parmi les amphocarboxyglycinates et les amphocarboxypropionates.
A titre d'exemples, on peut citer les composés classés dans le dictionnaire CTFA, 9ème édition, 2002, sous les dénominations Disodium Cocoamphodiacetate, Disodium Lauroamphodiacetate, Disodium Caproamphodiacetate, Disodium Capryloampho diacetate, Disodium Cocoamphodipropionate, Disodium Lauroamphodipropionate, Disodium Caproamphodipropionate, Disodium Capryloamphodipropionate, Lauroampho dipropionic acid, Cocoamphodipropionic acid, seuls ou en mélanges.

Parmi les tensioactifs non ioniques susceptibles d'être utilisés dans la composition selon l'invention, figurent notamment les alkylpyrrolidones non ioniques. Ces tensioactifs sont de préférence des alkyl(C1-C30)pyrrolidones. A titre d'exemples, on peut citer les composés classés dans le dictionnaire CTFA, 9ème édition, 2002, sous les dénominations Lauryl pyrrolidone, Caprylyl pyrrolidone, Methyl pyrrolidone, seuls ou en mélanges.

Les éthers d'alcools gras oxyalkylénés ou glycérolés sont plus particulièrement choisis parmi les alcools gras éventuellement hydroxylés linéaires ou ramifiés, saturés ou insaturés éthoxylés et/ou propoxylés et/ou glycérolés, ayant une chaîne grasse comportant par exemple de 8 à 30 atomes de carbone, le nombre de groupements oxyde d'éthylène et/ou oxyde de propylène pouvant aller notamment de 1 à 200 et le nombre de groupements glycérol pouvant aller notamment de 1 à 30.
Il peut s'agir de mono ou de diéthers.
A titre d'exemples on peut citer les composés classés dans le dictionnaire CTFA, 9ème édition, 2002, sous les dénominations Oleyl glyceryl ether, Oleth-2, Oleth-8, Oleth-106, Steareth-20, Laureth-10, PEG-4 ditallow ether, PPG- Beneth-15, PPG-8 Ceteth-1, PPG-50 Cetyl ether, PPG-6-Decyltetradeceth-12, PPG-2-Isodeceth-9,PPG-30 Isocethylether, PPG-4 Laurylether,PPG-10 Oleylether.
De préférence, les éthers d'alcools gras sont choisis parmi les alcools gras éventuellement hydroxylés linéaires ou ramifiés, saturés ou insaturés éthoxylés et propoxylés, ou propoxylés, et/ou glycérolés.

Les esters d'acides gras et de monoalcools oxyalkylénés ou glycérolés susceptibles d'être utilisés dans la composition de l'invention sont choisis de préférence parmi les esters d'acides carboxyliques linéaires ou ramifiés, saturés ou insaturés, ayant une chaîne grasse comportant par exemple de 8 à 30 atomes de carbone.
Dans le cas où il s'agit d'un ester d'acide gras et de monoalcool, ce dernier possède avantageusement une chaîne grasse comportant par exemple de 8 à 30 atomes de carbone, linéaire ou ramifiée, saturée ou insaturée, éthoxylée et/ou propoxylée ou glycérolée. Par ailleurs, le nombre de groupements oxyde d'éthylène ou oxyde de propylène de ce monoalcool peut être compris entre 1 et 200 et le nombre de groupements glycérol peut être compris entre 1 et 30.

A titre d'exemples de ces esters on peut citer les composés classés dans le dictionnaire CTFA, 9ème édition, 2002, sous les dénominations Steareth-12 stearate, Steareth-5 stearate.

La teneur en tensioactifs amphotères et/ou en tensioactifs non ioniques tels qu'ils viennent d'être définis, représente plus de manière avantageuse de 0,01 à 30 % en poids, plus particulièrement de 0,1 à 20 % en poids et de préférence de 0,2 à 10% en poids, du poids total de la composition.

La composition mise en oeuvre dans le cadre de l'invention comprend de plus un ou plusieurs colorants fluorescents.

Par colorant fluorescent, on entend au sens de la présente invention un colorant qui est une molécule qui colore par elle-même et donc absorbe la lumière du spectre visible et éventuellement de l'ultraviolet (longueurs d'onde allant de 360 à 760 nanomètres) mais qui, contrairement à un colorant classique, transforme l'énergie absorbée en lumière fluorescente de plus grande longueur d'onde émise dans la partie visible du spectre.

Le colorant fluorescent présent dans la composition selon l'invention est à différencier d'un agent éclaircissant optique. Les agents éclaircissants optiques appelés généralement azurants optiques, ou "brighteners", ou "fluorescent brighteners", ou "fluorescent brightening agents", ou "fluorescent whitening agents", ou "whiteners", ou encore "fluorescent whiteners" en terminologie anglo-saxone, sont des composés transparents incolores. Ces composés ne colorent pas car ils n'absorbent pas dans la lumière visible, mais uniquement dans les ultraviolets (longueurs d'onde allant de 200 à 400 nanomètres). Ils transforment l'énergie absorbée en lumière fluorescente de plus grande longueur d'onde émise dans la partie visible du spectre ; l'impression de couleur est alors uniquement engendrée par la lumière purement fluorescente à prédominante bleue (longueurs d'onde allant de 400 à 500 nanomètres).

Enfin, le colorant fluorescent mis en oeuvre dans la composition est soluble dans le milieu de la composition. Précisons que le colorant fluorescent est différent en cela d'un pigment fluorescent qui lui, n'est pas soluble dans le milieu de la composition.

Plus particulièrement, le colorant fluorescent utilisé dans le cadre de la présente invention, éventuellement neutralisé, est soluble dans le milieu de la composition à au moins 0,001 g/l, plus particulièrement au moins 0,5 g/l, de préférence au moins 1 g/l et selon un mode de réalisation encore plus préféré, au moins 5 g/l à la température comprise entre 15 et 25°C.

Les colorants fluorescents utilisés selon la présente invention sont des colorants dans la gamme des orangés.

Selon un mode de réalisation particulier de l'invention, les colorants fluorescents de l'invention conduisent à un maximum de réflectance se situant dans la gamme de longueur d'onde allant de 500 à 650 nanomètres, et de préférence dans la gamme de longueur d'onde allant de 550 à 620 nanomètres

Certains des colorants fluorescents selon la présente invention sont des composés connus en eux-mêmes.

A titre d'exemples de colorants fluorescents mis en oeuvre, on peut citer les colorants fluorescents appartenant aux familles suivantes : les naphtalimides ; les coumarines cationiques ou non ; les xanthénodiquinolizines (comme notamment les sulforhodamines) ; les azaxanthènes ; les naphtolactames ; les azlactones ; les oxazines ; les thiazines ; les dioxazines ; les colorants fluorescents polycationiques de type azoïque, azométhinique, ou méthinique, seuls ou en mélanges, de préférence aux familles suivantes : les naphtalimides ; les coumarines cationiques ou non ; les azaxanthènes ; les naphtolactames ; les azlactones ; les oxazines ; les thiazines ; les dioxazines ; les colorants fluorescents polycationiques de type azoïque, azométhinique, ou méthinique, seuls ou en mélanges.

Parmi les colorants fluorescents solubles de ce type, on peut notamment citer:
- le Jaune Brilliant B6GL commercialisé par la société SANDOZ et de structure 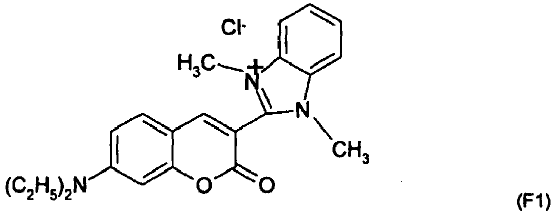
- le Basic Yellow 2, ou Auramine O commercialisé par les sociétés PROLABO, ALDRICH ou CARLO ERBA et de structure suivante : 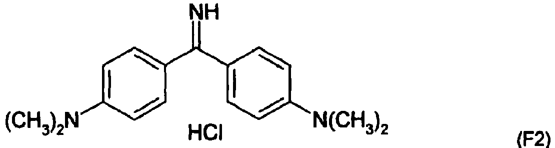 monochlorhydrate de 4,4'-(imidocarbonyl)bis(N,N-diméthylaniline) - CAS number 2465-27-2.

On peut aussi citer les composés de formule suivante :

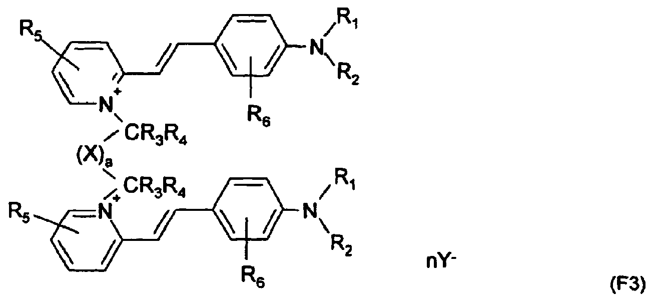

Dans cette formule, R₁, R₂, identiques ou différents, représentent :
- un atome d'hydrogène ;
- un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
- un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaire ou ramifié comprenant 1 à 4 atomes de carbone éventuellement interrompu et/ou substitué par au moins un hétéroatome et/ou groupement comprenant au moins un hétéroatome et/ou substitué par au moins un atome d'halogène ;
- R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
- R₁ ou R₂ peut éventuellement être engagé dans un hétérocycle comprenant l'atome d'azote et l'un des atomes de carbone du groupement phényle portant ledit atome d'azote.

Rappelons que les termes hétéroatomes, représentent un atome d'oxygène ou d'azote. Parmi les groupements porteurs de tels atomes, on peut citer entre autres les groupements hydroxyle, alcoxy, carbonyle, amino, ammonium, amido (-N-CO-), carboxyle (-O-CO- ou -CO-O-).
En ce qui concerne les groupements alcényles, ces derniers comprennent une ou plusieurs liaisons carbone-carbone insaturée (-C=C-), et de préférence une seule double liaison carbone-carbone.

Dans cette formule générale, les radicaux R₁ et R₂, identiques ou non, représentent plus particulièrement:
- un atome d'hydrogène ;
- un radical alkyle comprenant 1 à 10 atomes de carbone, notamment 1 à 6 atomes de carbone, de préférence 1 à 4 atomes de carbone, éventuellement interrompu par un atome d'oxygène ou éventuellement substitué par au moins un radical hydroxyle, amino, ammonium, d'un atome de chlore ou de fluor ;
- un radical benzyle, phényle, éventuellement substitué par un radical alkyle ou alcoxy comprenant 1 à 4 atomes de carbone, de préférence 1 ou 2 atomes de carbone ;
- avec l'atome d'azote, un radical hétérocylique du type pyrrolo, pyrrolidino, imidazolino, imidazolo, imidazolium, pyrazolino, pipérazino, morpholino, morpholo, pyrazolo, triazolo, éventuellement substitué par au moins un radical alkyle linéaire ou ramifié comprenant 1 à 4 atomes de carbone éventuellement interrompu et/ou substitué par un atome d'azote et/ou d'oxygène et/ou groupement portant un atome d'azote et/ou d'oxygène.

En ce qui concerne les radicaux amino ou ammonium précités, les radicaux portés par l'atome d'azote peuvent ou non être identiques et représenter plus particulièrement un atome d'hydrogène, un radical alkyle en C₁-C₁₀, de préférence en C₁-C₄, un radical arylalkyle dans lequel, plus spécialement, le radical aryle comprend 6 atomes de carbone et le radical alkyle 1 à 10 atomes de carbone, de préférence 1 à 4 atomes de carbone.

Selon un mode de réalisation avantageux de l'invention, les radicaux R₁ et R₂, identiques ou non, représentent un atome d'hydrogène ; un radical alkyle linéaire ou ramifié en C₁-C₆ ; un radical alkyle en C₂-C₆ substitué par un radical hydroxyle ; un radical alkyle en C₂-C₆ portant un groupement amino ou ammonium ; un radical chloroalkyle en C₂-C₆ ; un radical alkyle C₂-C₆ interrompu par un atome d'oxygène ou groupement en portant un (par exemple ester) ; un radical aromatique comme les phényle, benzyle, 4-méthylphényle ; un radical hétérocyclique tel que les radicaux pyrrolo, pyrrolidino, imidazolo, imidazolino, imidazolium, pipérazino, morpholo, morphollino, pyrazolo, triazolo, éventuellement substitué par au moins un radical alkyle en C₁-C₆ ou aromatique.

De préférence, les radicaux R₁ et R₂, identiques ou différents, représentent un atome d'hydrogène, un radical alkyle linéaire ou ramifié en C₁-C₆ tels que les radicaux méthyle, éthyle, n-butyle, n-propyle ; le 2-hydroxyéthyle ; un radical alkyltriméthylammonium ou alkyltriéthylammonium, le radical alkyle étant linéaire en C₂-C₆ ; un radical (di)alkylméthylamino ou (di)alkyléthylamino, le radical alkyle étant linéaire en C₂-C₆ ; - CH₂CH₂Cl ; -(CH2)ₙ-OCH₃ ou -(CH₂)ₙ-OCH₂CH₃ avec n nombre entier variant de 2 à 6 ; -CH₂CH₂-OCOCH₃ ; -CH₂CH₂COOCH₃.
De préférence les radicaux R₁ et R₂, identiques ou non, et de préférence identiques, représentent un radical méthyle, un radical éthyle.

Les radicaux R₁ et R₂, identiques ou différents, peuvent aussi représenter un radical hétérocyclique du type pyrrolidino, 3-amino pyrrolidino, 3-(diméthyl)amino pyrrolidino, 3-(triméthyl)amino pyrrolidino, 2,5-diméthylpyrrolo, le 1H-imidazole, 4-méthyl pipérazino, 4-benzyl pipérazino, morpholo, 3,5-(ter-butyl)-1H-pyrazolo, 1H-pyrazolo, 1H-1,2,4-triazolo.

Les radicaux R₁ et R₂, identiques ou différents, peuvent aussi représenter être reliés de manière à former un hétérocycle de formules (I) et (II) suivantes : 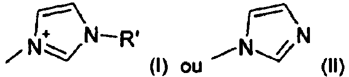 dans lesquelles R' représente un atome d'hydrogène, un radical alkyle en C₁-C₃,-CH₂CH₂OH, -CH₂CH₂OCH₃.

Conformément à un mode de réalisation plus particulier de l'invention, R₅, identiques ou non, représentent un atome d'hydrogène, un atome de fluor ou de chlore, un radical alkyle linéaire ou ramifié comprenant 1 à 4 atomes de carbone éventuellement interrompu par un atome d'oxygène ou d'azote.
Il est précisé que le substituant R₅, s'il est différent de l'hydrogène, se trouve avantageusement en position(s) 3 et/ou 5 par rapport au carbone du cycle portant l'azote substitué par les radicaux R₁ et R₂, et de préférence en position 3 par rapport à ce carbone.
Avantageusement, les radicaux R₅, identiques ou non, représentent un atome d'hydrogène ; un radical alkyle linéaire ou ramifié en C₁-C₄ ; -O-R₅₁ avec R₅₁ représentant un radical alkyle linéaire en C₁-C₄ ; -R₅₂-O-CH₃ avec R₅₂ représentant un radical alkyle linéaire en C₂-C₃ ; -R₅₃ - N (R₅₄)₂ dans laquelle R₅₃ représente un radical alkyle linéaire en C₂-C₃, R₅₄, identiques ou différents, représentent un atome d'hydrogène ou un radical méthyle.
De préférence R₅, identiques ou non, représentent l'hydrogène, un méthyle, un méthoxy, et de préférence, R₅ représente un atome d'hydrogène.

Selon un mode de réalisation particulier, les radicaux R₆, identiques ou différents, représentent un atome d'hydrogène ; un radical alkyle linéaire ou ramifié en C₁-C₄ ; -X avec X représentant un atome de chlore, de brome ou de fluor ; -R₆₁-O-R₆₂ avec R₆₁ représentant un radical alkyle linéaire en C₂-C₃ et R₆₂ représente le radical méthyle ; -R₆₃-N(R₆₄)₂ avec R₆₃ représentant un radical alkyle linéaire en C₂-C₃, R₆₄, identiques ou différents, représentent un atome d'hydrogène ou un radical méthyle, -N(R₆₅)₂ dans laquelle R₆₅, identiques ou différents, représentent un atome d'hydrogène, un radical alkyle linéaire en C₂-C₃ ; -NHCO R₆₆ avec R₆₆ représentant un radical alkyle en C₁-C₂, un radical chloroalkyle en C₁-C₂, un radical -R₆₇-NH₂ ou-R₆₇-NH(CH₃) ou -R₆₇-N(CH₃)₂ ou -R₆₇-N⁺(CH₃)₃ ou -R₆₇-N⁺(CH₂CH₃)₃ avec R₆₇ représentant un radical alkyle en C₁-C₂.
Il est précisé que le substituant R₆, s'il est différent de l'hydrogène, se trouve de préférence en position 2 et/ou 4 par rapport à l'atome d'azote du cycle pyridinium, et de préférence en position 4 par rapport à cet atome d'azote.

Plus particulièrement ces radicaux R₆, identiques ou non, représentent un atome d'hydrogène ou un radical méthyle ou éthyle, et de préférence, R₆ représente un atome d'hydrogène.

En ce qui concerne les radicaux R₃, R₄, ces derniers, identiques ou non, représentent avantageusement un atome d'hydrogène, un radical alkyle comprenant 1 à 4 atomes de carbone, plus spécialement un radical méthyle. De manière préférée, R₃ et R₄ représentent chacun un atome d'hydrogène.

Comme indiqué plus haut, X représente :
- un radical alkyle, linéaire ou ramifié comprenant 1 à 14 atomes de carbone, ou alcényle comprenant 2 à 14 atomes de carbone, éventuellement interrompu et/ou substitué par au moins un hétéroatome, par au moins un groupement porteur d'au moins un hétéroatome et/ou par au moins un atome d'halogène ;
- un radical hétérocyclique comprenant 5 ou 6 chaînons, éventuellement substitué par au moins un radical alkyle linéaire ou ramifié comprenant 1 à 14 atomes de carbone, par au moins un radical aminoalkyle, linéaire ou ramifié, comprenant 1 à 4 atomes de carbone, éventuellement substitué par au moins un hétéroatome ; par au moins un atome d'halogène ;
- un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement comprenant au moins un hétéroatome ;
- un radical dicarbonyle.
En outre, il est indiqué que le groupement X peut porter une ou plusieurs charges cationiques.

Ainsi, X peut représenter un radical alkyle, linéaire ou ramifié, comprenant 1 à 14 atomes de carbone, ou alcényle comprenant 2 à 14 atomes de carbone, et peut être substitué et/ou interrompu par un ou plusieurs atomes d'oxygène et/ou d'azote, et/ou par un ou plusieurs groupements porteurs d'au moins un hétéroatome , et/ou par un atome de fluor, de chlore.
Parmi les groupements de ce type, on peut citer tout particulièrement les groupements hydroxyle, alcoxy (avec notamment un radical R de type alkyle en C₁-C₄), amino, ammonium, amido, carbonyle, carboxyle (-COO-, -O-CO-) avec notamment un radical de type alkyloxy.
Notons que l'atome d'azote, s'il est présent, peut se trouver sous une forme quaternisée ou non. Dans ce cas, le ou les deux autres radicaux portés par l'atome d'azote quaternisé ou non, sont identiques ou non et peuvent être un atome d'hydrogène, un radical alkyle en C₁-C₄, de préférence le méthyle.

Selon une autre variante, le groupement X représente un radical hétérocyclique comprenant 5 ou 6 chaînons, du type imidazolo, pyrazolo, triazino, pyridino, éventuellement substitué par au moins un radical alkyle linéaire ou ramifié comprenant 1 à 14 atomes de carbone, plus particulièrement 1 à 10 atomes de carbone, de préférence de 1 à 4 atomes de carbone ; par au moins un radical aminoalkyle, linéaire ou ramifié, comprenant 1 à 10 atomes de carbone, de préférence de 1 à 4 atomes de carbone, éventuellement substitué par un groupement comprenant au moins un hétéroatome (de préférence un radical hydroxyle), ou par un atome d'halogène. A noter que le groupement amino est de préférence lié à l'hétérocycle.

Conformément à une autre possibilité, le groupement X représente un radical aromatique (comprenant de préférence 6 atomes de carbone) ou diaromatique condensé ou non (comprenant notamment de 10 à 12 atomes de carbone), séparé ou non par un radical alkyle comprenant 1 à 4 atomes de carbone, le ou les radicaux aryles étant éventuellement substitués par au moins au moins un atome d'halogène et/ou par au moins un radical alkyle comprenant 1 à 10 atomes de carbone, de préférence 1 à 4 atomes de carbone, éventuellement interrompu par au moins un atome d'oxygène et/ou d'azote, et/ou groupement comprenant au moins un hétéroatome (comme un radical carbonyle, carboxyle, amido, amino, ammonium).
Il est à noter que le radical aromatique, de préférence un radical phényle, est relié aux groupements CR₃R₄ par l'intermédiaire de liaisons en positions 1,2 ; 1,3 ou 1;4, de préférence en positions 1,3 et 1,4. Si le radical phényle relié par l'intermédiaire de liaisons en positions 1,4, porte un ou deux substituants, ce ou ces derniers sont situés de préférence en position 1,4 par rapport à l'un des groupements CR₃R₄. Si le radical phényle relié par l'intermédiaire de liaisons en positions 1,3, porte un ou deux substituants, ce ou ces derniers sont situés de préférence en position 1 et/ou 3 par rapport à l'un des groupements CR₃R₄.

Au cas où le radical est diaromatique, il est de préférence non condensé et comprend deux radicaux phényles séparés ou non par une liaison simple (soit un carbone de chacun des deux cycles) ou par un radical alkyle, de préférence de type CH₂ ou C(CH₃)₂. De manière préférée, les radicaux aromatiques ne portent pas de substituant.
Il est à noter que ledit radical diaromatique est relié aux groupements CR₃R₄ par l'intermédiaire de liaisons en positions 4,4'.

A titre d'exemples de groupements X convenables, on peut citer notamment les radicaux alkyle linéaires ou ramifiés comprenant 1 à 13 atomes de carbone tels que méthylène, éthylène, propylène, isopropylène, n-butylène, pentylène, hexylène ; le 2-hydroxypropylène, le 2-hydroxy n-butylène ; les radicaux alkylènes en C₁-C₁₃, substitués ou interrompus par un ou plusieurs atomes d'azote et/ou d'oxygène, et/ou groupements portant au moins un hétéroatome (hyroxyle, amino, ammonium, carbonyle, carboxyle, par exemple) tels que -CH₂CH₂OCH₂CH₂-, le 1,6-didéoxy-d-mannitol, -CH₂N⁺(CH₃)₂CH₂-, -CH₂CH₂N⁺(CH₃)₂-(CH₂)₆N⁺(CH₃)₂-CH₂CH₂-, CO-CO-, le 3,3-diméthylpentylène, le 2-acétoxyéthylène, le butylène1,2,3,4 tétraol ; -CH=CH- ; les radicaux aromatiques ou diaromatiques substitués par un ou plusieurs radicaux alkyle, par un ou plusieurs groupements portant au moins un hétéroatome et/ou par un ou plusieurs atomes d'halogène, tels que le 1,4-phénylène, le 1,3-phénylène, le 1,2-phénylène, le 2,6-fluorobenzène, le 4,4'-biphénylène, le 1,3-(5-méthyl benzène), le 1,2-bis(2-méthoxy)benzène, le bis(4-phényl)méthane, le 3,4 benzoate de méthyle, le 1,4-bis(amido méthyl)phényle; les radicaux de type hétérocycliques comme la pyridine, ou dérivé tel que le 2,6-bispyridine, l'imidazole, l'imidazolium, la triazine.

X représente, selon un mode de réalisation plus particulier de l'invention, un radical alkyle linéaire ou ramifié en C₁-C₁₃ ; -CH₂CH(OH)CH₂- ; -CH₂CH(Cl)CH₂- ;-CH₂CH₂-OCOCH₂- ; -CH₂CH₂COOCH₂- ; -Ra-O- Rb- avec Ra représentant un radical alkyle linéaire en C₂-C₆ et Rb représente un radical alkyle linéaire en C₁-C₂ ; - Rc-N(Rd)-Re- avec Rc représentant un radical alkyle en C₂-C₉, Rd représentant un atome d'hydrogène, un radical alkyle en C₁-C₂ et Re représentant un radical alkyle en C₁-C₆ ; - Rf-N⁺(R_{g})₂-Rh- avec Rf représentant un radical alkyle linéaire en C₂-C₉, Rg, de préférence identiques, représentent un radical alkyle en C₁-C₂, Rh représente un radical alkyle linéaire en C₁-C₆ ; -CO-CO-.

X peut de plus représenter un radical imidazole, éventuellement substitué par au moins un radical alkyle comprenant 1 à 14 atomes de carbone, plus particulièrement 1 à 10 atomes de carbone, de préférence de 1 à 4, et par exemple les radicaux divalents de formule suivante : 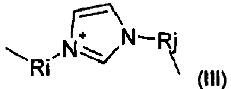 dans laquelle Ri et Rj, identiques ou non, représentent un radical alkyle linéaire en C₁-C₆

X peut de même être choisi parmi les radicaux divalents dérivés de triazine suivants :

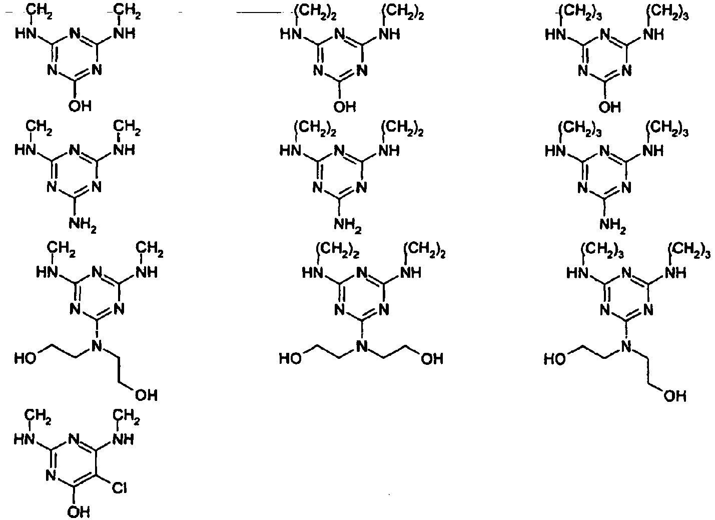

Selon une autre possibilité, X peut représenter les radicaux divalents aromatiques suivants :

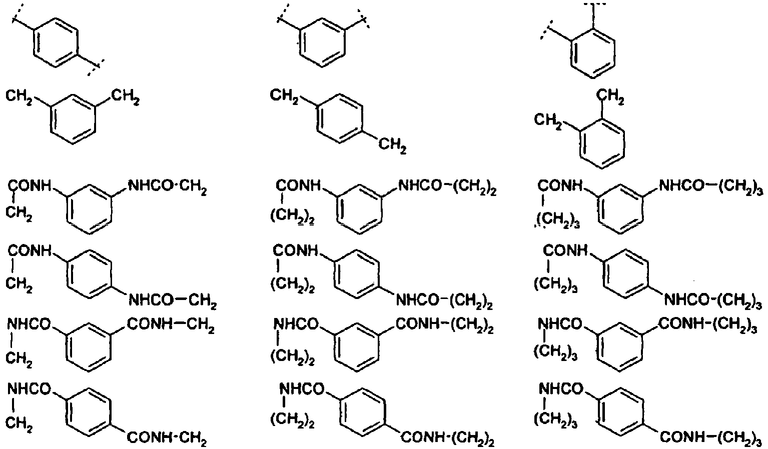

Dans la formule générale de ces composés fluorescents, Y⁻ représente un anion organique ou minéral. S'il y a plusieurs anions Y⁻, ces derniers peuvent ou non être identiques.

Parmi les anions d'origine minérale, on peut citer sans intention de s'y limiter les anions provenant d'atomes d'halogène, tels que les chlorures de préférence, les iodures, les sulfates ou bisulfates, les nitrates, les phosphates, les hydrogénophosphates, les dihydrogénophosphates, les carbonate, les bicarbonates.
Parmi les anions d'origine organique, on peut citer les anions provenant des sels d'acides mono- ou polycarboxyliques, sulfoniques, sulfuriques, saturés ou non, aromatiques ou non, éventuellement substitués par au moins un radical hydroxyle, amino, ou atomes d'halogène. A titre d'exemples non limitatifs, conviennent les acétates, hydroxyactétates, aminoacétates, (tri)chloroacétates, benzoxyactétates, propionates et dérivés portant un atome de chlore, fumarates, oxalates, acrylates, malonates, succinates, lactates, tartrates, glycollates citrates, les benzoates et dérivés portant un radical méthyle ou amino, les alkylsulfates, les tosylates, les benzènesulfonates, toluènesulfonates, etc.
De préférence, le ou les anions Y, identiques ou non, sont choisis parmi le chlore, le sulfate, le méthosulfate, l'éthosulfate.

Enfin, le nombre n, entier, est au moins égal à 2 et au plus égal au nombre de charges cationiques présentes dans le composé fluorescent.

De préférence les composés fluorescents qui viennent d'être détaillés sont des composés symétriques.

Ces composés peuvent être synthétisés en mettant en faisant réagir dans une première étape de α-picoline avec un réactif comprenant deux groupes partant qui peuvent être choisis parmi les atomes d'halogène, de préférence le brome, éventuellement le chlore, ou les groupements de type tolylsulfonyle ou méthylesulfonyle.
Cette première étape peut avoir lieu en présence d'un solvant, bien qu'il ne soit pas obligatoire, comme par exemple le diméthylformamide.
Le nombre de moles d'α-picoline est en général voisin de 2 pour une mole de réactif comprenant les groupes partant.
En outre, la réaction est habituellement mise en oeuvre au reflux du réactif et/ou du solvant s'il est présent.
Le produit issu de cette première étape est ensuite contacté avec un aldéhyde correspondant de formule suivante : 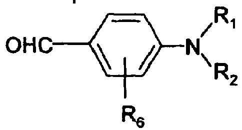 dans laquelle R₁, R₂ et R₆ ont les mêmes significations que précédemment indiquées. Là encore, la réaction peut être effectuée en présence d'un solvant approprié, de préférence au reflux.
Il est à noter que les radicaux R₁ et R₂ de l'aldéhyde peuvent avoir la signification indiquée dans la formule générale détaillée auparavant.
Il est aussi possible de mettre en oeuvre un aldéhyde pour lequel lesdits radicaux représentent des atomes d'hydrogène et effectuer conformément à des méthodes classiques, la substitution de ces atomes d'hydrogène par des radicaux appropriés tels que décrits dans la formule générale une fois la deuxième étape terminée.

On pourra notamment se référer à des synthèses telles que décrites dans US 4256458.

Il est précisé que conformément à un mode de réalisation particulier de l'invention, la composition ne comprend pas, à titre de colorant fluorescent, un composé comprenant trois noyaux aromatiques condensés dont l'un comprend un atome d'oxygène.

Le ou les colorants fluorescents de la présente invention représentent plus habituellement de 0,01 à 20 % en poids, plus particulièrement de 0,05 à 10 % en poids et de préférence de 0,1 à 5% en poids, du poids total de la composition.

Le milieu cosmétiquement acceptable est généralement constitué par de l'eau ou par un mélange d'eau et d'au moins un solvant organique.

Parmi les solvants convenables, on peut citer plus particulièrement, les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore les polyols comme le glycérol. On peut également utiliser comme solvant les polyéthylèneglycols et les polypropylèneglycols, et les mélanges de tous ces composés.

Les solvants peuvent être présents dans des proportions allant généralement de 1 à 40 % en poids, et de préférence de 5 à 30 % en poids, par rapport au poids total de la composition.

Le pH de la composition est généralement compris entre 3 et 12 environ, et de préférence entre 5 et 11 environ. Il peut être ajusté à la valeur désirée au moyen d'agents acidifiants ou alcalinisants.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 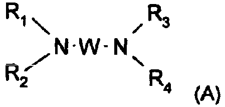 dans laquelle W est un reste propylène éventuellement substitué par un groupement hydroxyle ou un radical alkyle en C₁-C₆ ; R₁, R₂, R₃ et R₄, identiques ou différents, représentent un atome d'hydrogène, un radical alkyle en C₁-C₆ ou hydroxyalkyle en C₁-C₆.

La composition mise en oeuvre dans l'invention peut, selon une forme de réalisation préférée, et en plus du ou des colorants fluorescents, comprendre un ou plusieurs colorants directs additionnels non fluorescents de nature non ionique, cationique ou anionique.
Ces colorants directs additionnels peuvent par exemple être choisis parmi les colorants benzéniques nitrés, et plus particulièrement parmi les colorants benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylénediamine,
- le 1-amino-2-nitro-4-(β-hydroxyéthyl)amino-5-chloro benzène,
- la 2-nitro-4-amino-diphénylamine,
- le 1-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

La composition utilisable dans le cadre de l'invention peut également comprendre, en addition ou en remplacement de ces colorants benzéniques nitrés, un ou plusieurs colorants directs additionnels choisis parmi les colorants benzéniques nitrés jaunes, jaune-vert, bleus ou violets, les colorants azoïques, les colorants anthraquinoniques, naphtoquinoniques ou benzoquinoniques, les colorants indigoïdes, ou les colorants dérivés du triarylméthane.

Ces colorants directs additionnels peuvent notamment être des colorants basiques parmi lesquels on peut citer plus particulièrement les colorants connus dans le COLOR INDEX, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le COLOR INDEX, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans les demandes de brevet WO 95/01772, WO 95/15144 et EP-A-0 714 954 et dont le contenu fait partie intégrante de la présente invention.

Parmi les colorants directs additionnels benzéniques nitrés jaunes et jaune-vert, on peut par exemple citer les composés choisis parmi :
- le 1-β-hydroxyéthyloxy-3-méthylamino-4-nitrobenzéne,
- le 1-méthylamino-2-nitro-5-(β,γ-dihydroxypropyl)oxy benzène,
- le 1-(β-hydroxyéthyl)amino-2-méthoxy-4-nitrobenzéne,
- le 1-(β-aminoéthyl)amino-2-niro-5-méthoxy-benzéne,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzéne,
- le 4-(β,γ-dihydroxypropyl)amino-3-nitro-trifluorométhylbenzène,
- le 1-(β-uréidoéthyl)amino-4-nitrobenzéne,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzéne,
- le 4-(β-hydroxyéthyl)amino-3-nitrobenzamide.

Parmi les colorants directs additionnels benzéniques nitrés bleus ou violets, on peut par exemple citer les composés choisis parmi :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-(β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 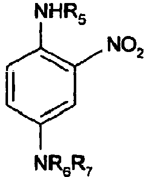 dans laquelle :
   ∘ R₆ représente un radical alkyle en C₁-C₄, un radical β-hydroxyéthyle ou β-hydroxypropyle ou γ-hydroxypropyle ;
   ∘ R₅ et R₇, identiques ou différents, représentent un radical β-hydroxyéthyle, -β-hydroxypropyle, γ-hydroxypropyle, ou β,γ-dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans le brevet français FR 2 692 572.

Lorsqu'ils sont présents, le ou les colorants directs additionnels représentent plus spécialement de 0,0005 à 12 % en poids, de préférence, de 0,005 à 6 % en poids, du poids total de la composition.

La composition peut également comprendre divers adjuvants utilisés, tels que des polymères anioniques, cationiques, non ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des cations, des polymères cationiques ou amphotères, des silicones volatiles ou non volatiles, modifiées ou non modifiées, des agents filmogènes, des céramides, des agents conservateurs, des agents stabilisants, des agents opacifiants.

Parmi les agents épaississants, on préfère plus particulièrement utiliser les systèmes épaississants à base de polymères associatifs bien connus de l'homme de l'art et notamment de nature non ionique, anionique, cationique ou amphotère.

Bien entendu, l'homme de l'art veillera à choisir ce ou ces éventuels composés complémentaires de manière telle que les propriétés avantageuses attachées intrinsèquement à la composition ne soient pas, ou substantiellement pas, altérées par la ou les adjonctions envisagées.
Enfin, la composition utilisée dans le carde de l'invention peut se présenter sous des formes diverses, telles que sous forme de liquides, de shampooings, de crèmes, de gels, ou sous toute autre forme appropriée.

Selon un mode de réalisation préféré, la composition se trouve sous la forme d'un shampooing colorant et éclaircissant.

En outre, la présente invention est particulièrement adaptée au traitement de cheveux, pigmentés ou colorés artificiellement, dont la hauteur de ton est inférieure ou égale à 6 (blond foncé) et de préférence inférieure ou égale à 4 (châtain).
Dans ce qui suit, le terme cheveux sera utilisé indifféremment pour les cheveux mais aussi le système pileux (les cils, sourcils, etc.).
L'éclaircissement des cheveux est évalué par la "hauteur de ton" qui caractérise le degré ou le niveau d'éclaircissement. La notion de "ton" repose sur la classification des nuances naturelles, un ton séparant chaque nuance de celle qui la suit ou la précède immédiatement. Cette définition et la classification des nuances naturelles sont bien connues des professionnels de la coiffure et publiée dans l'ouvrage "Sciences des traitements capillaires" de Charles ZVIAK 1988, Ed.Masson, pp.215 et 278.
Les hauteurs de ton s'échelonnent de 1 (noir) à 10 (blond clair), une unité correspondant à un ton ; plus le chiffre est élevé et plus la nuance est claire.

Un procédé de coloration avec effet éclaircissant des cheveux représente un autre objet de la présente invention.

Il consiste dans une première étape à appliquer sur les cheveux une composition comprenant au moins colorant fluorescent et au moins un tensioactif amphotère et/ou non ionique, pendant un temps suffisant pour développer la coloration et l'éclaircissement désirés. Puis, éventuellement, dans une seconde étape, on rince éventuellement les cheveux. On peut éventuellement laver au shampooing les cheveux puis les rincer. Enfin, dans une dernière étape, on sèche ou on laisse sécher les cheveux.
Ainsi, le procédé selon l'invention peut consister à appliquer la composition pendant le temps nécessaire au développement de la coloration et de l'éclaircissement, puis à laisser sécher ou sécher les cheveux, sans rinçage final.

Le procédé de teinture et d'éclaircissement des cheveux est mis en oeuvre avec une composition telle que définie précédemment, en l'absence de colorants d'oxydation et d'agents oxydants.

Le temps nécessaire au développement de la coloration et à l'obtention de l'effet éclaircissant sur les cheveux est d'environ 5 à 60 minutes et plus particulièrement d'environ 5 à 40 minutes.

La température nécessaire au développement de la coloration et à l'obtention de l'effet éclaircissant sur les cheveux est généralement comprise entre la température ambiante (15 à 25°C) et 80°C et plus particulièrement entre 15 et 40°C.

Un autre objet de l'invention est constitué par l'utilisation d'une composition comprenant comprenant dans un milieu cosmétiquement acceptable, au moins un colorant fluorescent dans la gamme des orangés soluble dans le milieu et au moins un tensioactif amphotère choisi parmi les bétaïnes et les dérivés d'imidazolium et/ou au moins un tensioactif non ionique choisi parmi les alkylpyrrolidones, les éthers d'alcools gras oxyalkylénés ou glycérolés, les esters d'acides gras de monoalcools oxyalkylénés ou glycérolés, pour colorer avec un effet éclaircissant les fibres kératiniques humaines pigmentées ou colorées artificiellement, en l'absence de colorants d'oxydation et d'agents oxydants.

Dans le cadre de cette utilisation, le composé fluorescent est choisi parmi les composés fluorescents appartenant aux familles suivantes : naphtalimides ; les coumarines cationiques ou non ; les xanthénodiquinolizines (comme notamment les sulforhodamines) ; les azaxanthènes ; les naphtolactames ; les azlactones ; les oxazines ; les thiazines ; les dioxazines ; les colorants fluorescents monocationiques et polycationiques de type azoïque, azométhinique, ou méthinique, seuls ou en mélanges.

A titre de composés plus particuliers, on peut citer les composés de formules F1, F2 et F3 déjà détaillées auparavant.

On peut de même utiliser les composés de structure (F4) suivante : 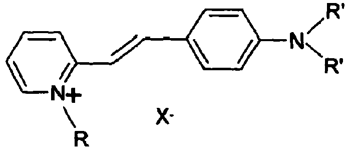 formule dans laquelle R représente un radical méthyle ou éthyle; R' représente un radical méthyle, X- un anion du type chlorure, iodure, sulfate, méthosulfate, acétate, perchlorate. A titre d'exemple de composé de ce type on peut citer le Photosensitiving Dye NK-557 commercialisé par la société UBICHEM, pour lequel R représente un radical éthyle, R' un radical méthyle et X- un iodure.

Tout ce qui a été décrit auparavant sur les natures et teneurs des divers additifs présents dans la composition reste valable et ne sera pas repris dans cette partie.

La présente invention permet d'obtenir des matières kératiniques traitées, et de manière particulièrement avantageuse des cheveux pigmentés ou artificiellement colorés, présentant une réflectance dans la gamme des longueurs d'onde allant de 500 à 700 nanomètres, de préférence de 540 à 700 nanomètres, supérieure à la réflectance des mêmes matières kératiniques non traitées conformément à l'invention.
Cela signifie que, dans la gamme de longueur d'onde allant de 500 à 700 nanomètres, de préférence de 540 à 700 nanomètres, il existe au moins une plage où la courbe de réflectance correspondant aux matières traitées, et plus particulièrement les cheveux, est supérieure à la courbe de réflectance correspondant aux matières non traitées.
On entend par "supérieure", un écart d'au moins 0,05% de réflectance, et de préférence d'au moins 0,1%.
Il est précisé toutefois qu'il peut exister dans la gamme de longueur d'onde allant de 500 à 700 nanomètres, de préférence de 540 à 700 nanomètres, une ou plusieurs plage où la courbe de réflectance correspondant aux matières traitées est soit superposable, soit inférieure à la courbe de réflectance correspondant aux matières non traitées.
De préférence, la longueur d'onde où l'écart est maximal entre la courbe de réflectance des matières traitées et celle non traitées, se situe dans la gamme de longueur d'onde allant de 500 à 650 nanomètres, et de préférence dans la gamme de longueur d'onde allant de 550 à 620 nanomètres.

De préférence l'effet d'éclaircissement est d'au moins 0,5 ton.

En outre, et de préférence, l'utilisation des compositions selon l'invention permet d'éclaircir les cheveux dans une nuance qui, chiffrée dans le système C.i.E.L L*a*b* présente une variable b* supérieure ou égale à 6, avec un rapport b*/valeur absolue de a*, supérieur à 1,2 selon le test de sélection décrit ci-dessous.

### Test de sélection

La composition est appliquée sur des cheveux châtains à raison de 10 grammes de composition pour 1 gramme de cheveux châtains. La composition est étalée de façon à recouvrir l'ensemble des cheveux. On laisse la composition agir pendant 20 minutes à température ambiante (20 à 25°C). Les cheveux sont ensuite rincés à l'eau puis lavés avec un shampooing à base de lauryléther sulfate. Ils sont ensuite séchés. On mesure alors les caractéristiques spectrocolorimétriques des cheveux pour en déterminer les coordonnées L*a*b*.
Dans le système C.I.E.L L*a*b*, a* et b* indiquent deux axes de couleurs, a* indique l'axe de couleur vert/rouge (+a* est rouge, -a* est vert) et b* l'axe de couleur bleu/jaune (+b* est jaune et - b* est bleu) ; des valeurs proches de zéro pour a* et b* correspondent à des nuances grises.

Les exemples qui suivent sont destinés à illustrer l'invention sans pour autant en limiter la portée.

### EXEMPLE 1

On a préparé les deux compositions suivantes :

| **Composition** | **1** | **2** |
|---|---|---|
| Colorant fluorescent NK-557 | 0,5% | 0,5% |
| N-cocoyl amidoéthyl N-éthoxycarboxyméthyl glycinate de sodium (Rhodia Chimie ; Miranol C 2M Conc.NP) | - | 2% M.A. |
| Eau distillée | q.s.p. 100 % | q.s.p. 100% |

La composition 2 fait partie de l'invention tandis que la composition 1 sert de référence.

Chacune des compositions est appliquée sur cheveux châtains (hauteur de ton 4) naturels pendant 20 minutes à température ambiante.
Le rapport de bain est fixé à 5.
A l'issue, les mèches sont rincées et séchées.

Les mèches sont lues au spectrofluorimètre Varian Cary Eclipse muni d'une fibre optique (excitation 480 nm, émission 580 nm, largeur de bande 5 nm). 8 mesures sont réalisées le long de la mèche.
Les résultats sont rassemblés ci-dessous :

| | Cheveux HT4 non traités | Cheveux HT4 + composition 1 | Cheveux HT4 + composition 2 |
|---|---|---|---|
| 1^{ère} mesure | 0,26 | 55,24 | 59,68 |
| 2^{ème} mesure | 0,17 | 46,69 | 61,43 |
| 3^{ème} mesure | 0,26 | 50,35 | 58,05 |
| 4^{ème} mesure | 0,25 | 51,47 | 59,98 |
| 5^{ème} mesure | 0,34 | 52,77 | 57,32 |
| 6^{ème} mesure | 0,29 | 44,61 | 60,52 |
| 7^{ème} mesure | 0,29 | 44,32 | 59,77 |
| 8^{ème} mesure | 0,37 | 46,68 | 54,75 |
| Moyenne | 0.28 | 49,01 | 58,94 |
| Ecart type | 0,06 | 4,02 | 2,14 |
| Intervalle de confiance (5%) | 0.04 | 2,78 | 1,48 |

On constate donc que la composition de l'invention conduit à une fluorescence plus importante.

### EXEMPLE 2

### Composé fluorescent

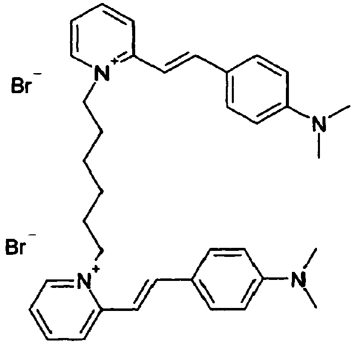

On fait réagir 93 g de 2-picoline avec 120g de 1,6 dibromohexane dans le diméthylformamide à 110°C pendant 5 heures.
On récupère le produit précipité, et on le filtre.
On solubilise 109 g du produit obtenu précédemment dans du méthanol et l'on ajoute 82,82 g de p-diméthylaminobenzaldéhyde en deux fois, en présence de pyrrolidine.
On laisse ensuite pendant 30 minutes.
On récupère le produit sous forme précipitée.
Analyse par spectroscopie de masse : 266.
Analyse élémentaire : C : 62,43 % ; H : 6,40 % ; Br : 23,07 % ; N : 8,09 %.
La formule est la suivante C₃₆H₄₄N₄.2Br.

### Composition

On a préparé la composition suivante :

| | |
|---|---|
| Composé fluorescent | 0,6 % |
| N-cocoyl amidoéthyl N-éthoxycarboxyméthyl glycinate de sodium | 2% |
| Hexylèneglycol | 7 % |
| Eau distillée qsp | 100 g |

Les pourcentages sont exprimés en poids de matière active.

### Coloration

La composition est appliquée sur une mèche de châtains naturels (hauteur de ton 4) avec un temps de pose de 20 minutes.
Les mèches sont ensuite rincées et un séchées au casque pendant 30 minutes.

On obtient un net effet d'éclaircissement de la mèche ainsi traitée.

## Revendications

1. Utilisation d'une composition comprenant dans un milieu cosmétiquement acceptable, au moins un colorant fluorescent dans la gamme des orangés soluble dans le milieu choisi parmi les colorants fluorescents appartenant aux familles suivantes : les naphtalimides ; les coumannes cationiques ou non ; les xanthénodiquinolizines ; les azaxanthènes ; les naphtolactames , les azlactones ; les oxazines ; les thiazines ; les dioxazines ; les colorants fluorescents monocationiques ou polycationiques de type azoïque, azométhinique, ou méthinique et au moins un tensioactif amphotère choisi parmi les bétaïnes et les dérivés d'imidazolium et/ou au moins un tensioactif non ionique choisi parmi les alkylpyrrolidones, les éthers d'alcools gras oxyalkylénés ou glycérolés, les esters d'acides gras de monoalcools oxyalkylénés ou glycérolés, pour colorer avec un effet éclaircissant des fibres kératiniques humaines pigmentées ou colorées artificiellement, en l'absence de colorants d'oxydation et d'agents oxydants.

2. Utilisation selon l'une des revendications 1 ou 2, **caractérisé en ce que** lesdites fibres présentent une hauteur de ton inférieure ou égale à 6, de préférence inférieure ou égale à 4.

3. Utilisation selon l'une quelconque des revendications 1 à 3, pour colorer avec un effet éclaircissant les cheveux avec un effet éclaircissant d'au moins 0,5 ton

4. Utilisation selon l'une quelconque des revendications précédentes, **caractérisée en ce que** les tensioactifs amphotères de type bétaïne sont choisis parmi les alkyl(C₈-C₂₀)bétaïnes, les alkyl(C₈-C₂₀)amidoalkyl(C₁-C₈)bétaïnes, les alkyl(C₈-C₂₀) amidoalkyl(C₁-C₈)sulfobétaines, les sulfobétaïnes ou leurs mélanges

5. Utilisation selon l'une quelconque des revendications 1 à 3, **caractérisée en ce que** les dérivés d'imidazolium sont choisis parmi les amphocarboxyglycinates et les amphocarboxypropionates, ou leurs mélanges.

6. Utilisation selon l'une quelconque des revendications 1 à 3, **caractérisée en ce que** les alkylpyrrolidones non ioniques sont des alkyl(C₁-C₃₀)pyrrolidones.

7. Utilisation selon l'une quelconque des revendications 1 à 3, **caractérisée en ce que** les éthers d'alcools gras oxyalkylénés ou glycérolés sont choisis parmi les alcools gras éventuellement hydroxylés, linéaires ou ramifiés, saturés ou insaturés, éthoxylés et/ou propoxylés ou glycérolés, ayant une chaîne comportant de 8 à 30 atomes de carbone.

8. Utilisation selon l'une quelconque des revendications 1 à 3, **caractérisée en ce que** les esters d'acides gras et de monoalcools oxyalkylénés ou glycérolés sont choisis parmi les esters d'acides carboxyliques, linéaires ou ramifiés, saturés ou insaturés ayant une chaîne grasse comportant de 8 à 30 atomes de carbone et d'un ou plusieurs monoalcools ayant une chaîne grasse comportant de 8 à 30 atomes de carbone, linéaire ou ramifiée, saturée ou insaturée éthoxylée et/ou propoxylée ou glycérolée.

9. Utilisation selon l'une quelconque des revendications précédentes, **caractérisée en ce que** le ou les tensioactifs amphotères et/ou le ou les tensioactifs non ioniques représentent de 0,01 à 30 % en poids, plus particulièrement de 0,1 à 20 % en poids et de préférence de 0,2 à 10% en poids du poids total de la composition.

10. Utilisation selon l'une quelconque des revendications précédentes, **caractérisée en ce que** le colorant fluorescent, éventuellement neutralisé, est soluble dans le milieu cosmétiquement acceptable à au moins 0,001 g/l, plus particulièrement au moins 0,5 g/l, de préférence au moins 1 g/l et selon un mode de réalisation encore plus préféré, au moins 5 g/l à la température comprise entre 15 et 25°C.

11. Utilisation selon l'une quelconque des revendications précédentes, **caractérisée en ce que** le colorant fluorescent conduit à un maximum de réflectance se situant dans la gamme de longueur d'onde allant de 500 à 650 nanomètres, et de préférence dans la gamme de longueur d'onde allant de 550 à 620 nanomètres.

12. Utilisation selon l'une quelconque des revendications précédentes, **caractérisée en ce que** le colorant fluorescent est choisi dans le groupe formé par les colorants de structures suivantes : 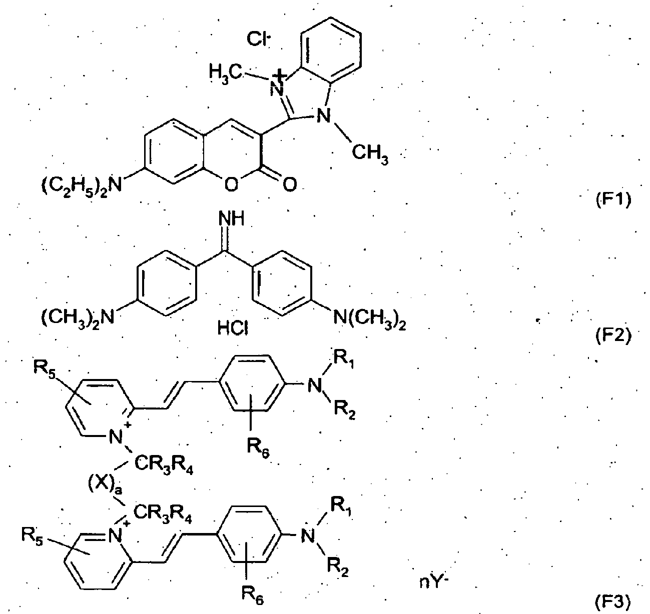 dans laquelle :
R₁, R₂, identiques ou différents, représentent :
• un atome d'hydrogène ;
• un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
• un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
• R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
• R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
• un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
• un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
• un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
• un radical dicarbonyle ;
• le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent ; 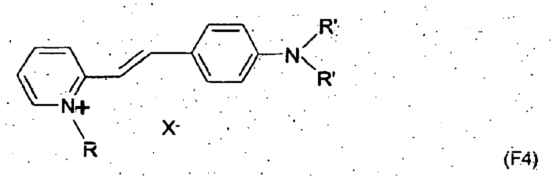 formule dans laquelle R représente un radical méthyle ou éthyle; R' représente un radical méthyle, X- un anion du type chlorure, iodure, sulfate, méthosulfate, acétate, perchlorate.

13. Utilisation selon l'une quelconque des revendications précédentes, **caractérisée par le fait que** le ou les colorants fluorescents sont présents dans une concentration pondérale allant de 0,01 à 20% en poids, plus particulièrement de 0,05 à 10% en poids, de préférence de 0,1 à 5% en poids, par rapport au poids total de la composition.

14. Utilisation selon l'une quelconque des revendications précédentes, **caractérisée en ce que** la composition comprend en outre au moins un colorant direct additionnel non fluorescent de nature non ionique, cationique ou anionique.

15. Utilisation selon la revendication 14, **caractérisée en ce que** les colorants directs additionnels sont choisis parmi les colorants benzéniques nitrés, les colorants azoïques, les colorants anthraquinoniques, naphtoquinoniques ou benzoquinoniques, les colorants indigoïdes, ou les colorants dérivés du triarylméthane, ou leurs mélanges.

16. Utilisation selon l'une quelconque des revendications **14** ou **15, caractérisée en ce que** le ou les colorants directs additionnels représentent de 0,0005 à 12 % en poids, de préférence de 0,005 à 6 % en poids, par rapport au poids total de la composition.

17. Utilisation selon l'une quelconque des revendications précédentes, **caractérisée en ce que** la composition se présente sous la forme d'un shampooing éclaircissant et colorant.

18. Utilisation selon l'une quelconque des revendications précédentes, **caractérisée en ce que** l'on met en œuvre les étapes suivantes
a) on applique sur les fibres kératiniques, la composition comprenant au moins colorant fluorescent et au moins un tensioactif amphotère et/ou non ionique, pendant un temps suffisant pour développer la coloration et l'éclaircissement désirés,
b) on rince éventuellement lesdites fibres,
c) éventuellement on lave au shampooing et on rince lesdites fibres,
d) on sèche ou on laisse sécher les fibres.

19. Utilisation selon l'une quelconque des revendications 1 à 4-615, **caractérisée en ce que** l'on applique sur la peau, la composition comprenant au moins colorant fluorescent et au moins un tensioactif amphotère et/ou non ionique, puis on sèche ou on laisse sécher la peau.

20. Composition **caractérisée en ce qu'elle** comprend, dans un milieu cosmétiquement acceptable, au moins un colorant fluorescent soluble dans le milieu choisi dans le groupe formé par les colorants de structures suivantes : 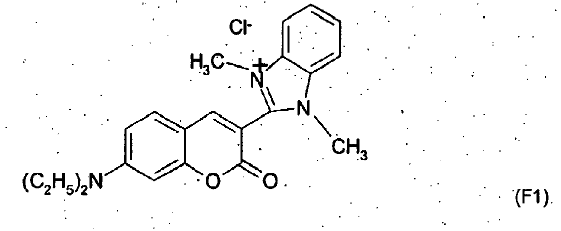 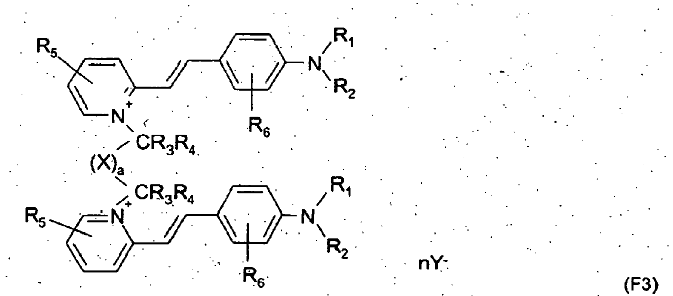 dans laquelle :
R₁, R₂, identiques ou différents, représentent :
• un atome d'hydrogène ;
• un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
• un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
• R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
• R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène,
un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ;
un radical alkyle linéaire ou ramifié comprenant 1 à 4 atomes de carbone,
éventuellement substitué et/ou interrompu par au moins un hétéroatome et/ou groupement portant au moins un hétéroatome et/ou substitué par au moins un atome d'halogène ;
X représente :
• un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
• un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
• un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
• un radical dicarbonyle ;
• le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent la composition ne comprenant pas, à titre d'agent fluorescent, du 2-[2-(4-dialkylamino)phényl éthényl]-1 alkyl pyridinium dans laquelle le radical alkyle du noyau pyridinium représente un radical méthyle, éthyle, celui du noyau benzénique représente un radical méthyle et dans laquelle le contre ion est un halogénure ; et au moins un tensioactif amphotère choisi parmi les bétaines et les dérivés d'imidazolium et/ou au moins un tensioactif non ionique choisi parmi les alkylpyrrolidones, les éthers d'alcools gras oxyalkylénés ou glycérolés, les esters d'acides gras de monoalcools oxyalkylénés ou glycérolés et les esters d'acides gras et de polyols,
en l'absence de colorants d'oxydation et d'agents oxydants.

21. Composition selon la revendication précédente, **caractérisée en ce que** le colorant fluorescent, éventuellement neutralisé, est soluble dans le milieu de la composition à au moins 0,001 g/l, plus particulièrement au moins 0,5 g/l, de préférence au moins 1 g/l et selon un mode de réalisation encore plus préféré, au moins 5 g/l à la température comprise entre 15 et 25°C.

22. Composition selon la revendication 20, **caractérisée en ce que** le colorant fluorescent est un colorant dans la gamme des orangés.

23. Composition selon l'une quelconque des revendications 20 à 21, **caractérisée en ce que** le colorant fluorescent conduit à un maximum de réflectance se situant dans la gamme de longueur d'onde allant de 500 à 650 nanomètres, et de préférence dans la gamme de longueur d'onde allant de 550 à 620 nanomètres.

24. Composition selon l'une des revendications 20 à 23, **caractérisée en ce que** les tensioactifs amphotères de type bétaïne sont choisis parmi les alkyl(C8-C20)bétaines, les alkyl(C8-C20)amidoalkyl(C1-C8)bétaïnes, les alkyl(C8-C20) amidoalkyl(C1-C8)sulfobétaines, les sulfobétaïnes ou leurs mélanges.

25. Composition selon l'une des revendications 20 à 23, **caractérisée en ce que** les dérivés d'imidazolium sont choisis parmi les amphocarboxyglycinates et les amphocarboxypropionates ou leurs mélanges.

26. Composition selon l'une des revendications 20 à 24, **caractérisée en ce que** les alkylpyrrolidones non ioniques sont des alkyl(C1-C30)pyrrolidones.

27. Composition selon l'une des revendications 20 à 25, **caractérisée en ce que** les éthers d'alcools gras oxyalkylénés ou glycérolés sont choisis parmi les alcools gras éventuellement hydroxylés linéaires ou ramifiés, saturés ou insaturés éthoxylés et/ou propoxylés ou glycérolés, ayant une chaîne comportant de 8 à 30 atomes de carbone.

28. Composition selon l'une des revendications 20 à 26, **caractérisée en ce que** les esters d'acides gras et de monoalcools oxyalkylénés ou glycérolés sont choisis parmi les esters d'acides carboxyliques, linéaires ou ramifiés, saturés ou insaturés ayant une chaîne comportant de 8 à 30 atomes de carbone et d'un monoalcool ayant une chaîne comportant de 8 à 30 atomes de carbone, linéaire ou ramifiée, saturée ou insaturée éthoxylée et/ou propoxylée ou glycérolée.

29. Composition selon l'une des revendications 20 à 27, **caractérisée en ce que** le ou les polyols sont choisis parmi la glycérine, le sorbitol, le glucose, le méthyl glucose, l'anhydride de sorbitol, un polyéthylène glycol ou un polypropylène glycol, ou leurs mélanges.

30. Composition selon l'une quelconque des revendications 20 à 28, **caractérisée en ce que** le ou les tensioactifs amphotères et/ou les tensioactifs non ioniques représentent de 0,01 à 30 % en poids, plus particulièrement de 0,1 à 20 % en poids, et de préférence de 0,2 à 10% en poids, du poids total de la composition.

31. Composition selon l'une quelconque des revendications 20 à 29, **caractérisée en ce que** le ou les colorants fluorescents sont présents dans une concentration pondérale allant de 0,01 à 20% en poids, plus particulièrement de 0,05 a 10% en poids, de préférence de 0,1 à 5% en poids, par rapport au poids total de la composition.

32. Composition selon l'une quelconque des revendications 20 à 30, **caractérisée en ce qu'**elle comprend au moins un colorant direct additionnel non fluorescent de nature non ionique, cationique ou anionique.

33. Composition selon la revendication 31, **caractérisée en ce que** les colorants directs additionnels sont choisis parmi les colorants benzéniques nitrés, les colorants azoïques, anthraquinoniques, naphtoquinoniques, benzoquinoniques, phénotiaziniques, les colorants indigoïdes xanthéniques, phénanthridiniques, phtalocyanines, ainsi que les colorants dérivés du triarylméthane, seuls ou en mélanges.

34. Composition selon l'une quelconque des revendications 31 ou 32, **caractérisée en ce que** le ou les colorants directs additionnels représentent de 0,0005 à 12 % en poids, de préférence de 0,005 à 6 % en poids, du poids total de la composition.

35. Composition selon l'une quelconque des revendications 20 à 33, **caractérisée en ce qu'**elle se présente sous la forme d'un shampooing éclaircissant et colorant.

36. Procédé pour colorer avec un effet éclaircissant les fibres kératiniques humaines pigmentées ou colorées artificiellement, **caractérisé en ce que** l'on met en œuvre les étapes suivantes :
a) on applique sur lesdites fibres une composition telle que définie selon l'une quelconque des revendications 20 à 33 et 35, pendant un temps suffisant pour développer la coloration et l'éclaircissement désirés,
b) on rince éventuellement les fibres,
c) éventuellement on lave au shampooing et on rince les fibres,
d) on sèche ou on laisse sécher les fibres,.
en l'absence de colorants d'oxydation et d'agents oxydants.

## Patentansprüche

1. Verwendung einer Zusammensetzung, die in einem kosmetisch unbedenklichen Medium mindestens einen in dem Medium löslichen Fluoreszenzfarbstoff im Orangebereich, ausgewählt aus den Fluoreszenzfarbstoffen, die zu den folgenden Familien gehören: Naphthalimide; kationische oder nichtkationische Cumarine; Xanthenodichinolizine; Azaxanthene; Naphtholactame; Azlactone; Oxazine; Thiazine; Dioxazine; monokationische oder polykationische Fluoresenzfarbstoffe vom Azo-, Azomethin- oder Methin-Typ, und mindestens ein amphoteres Tensid, ausgewählt aus Betainen und Imidazoliumderivaten, und/oder mindestens ein nichtionisches Tensid, ausgewählt aus Alkylpyrrolidonen, oxyalkylenierten oder glycerinierten Fettalkoholethern, umfasst, zum aufhellend wirkenden Färben von pigmentierten oder künstlich gefärbten menschlichen Keratinfasern in Abwesenheit von Oxidationsfarbstoffen und Oxidationsmitteln.

2. Verwendung nach Anspruch 1 oder 2, **dadurch gekennzeichnet, dass** die Fasern eine Tonhöhe kleiner gleich 6 und vorzugsweise kleiner gleich 4 aufweisen.

3. Verwendung nach einem der Ansprüche 1 bis 3 zum aufhellend wirkenden Färben der Haare mit einer Aufhellungswirkung von mindestens 0,5 Ton.

4. Verwendung nach einem der vorhergehenden Ansprüche, **dadurch gekennzeichnet, dass** die amphoteren Tenside vom Betain-Typ aus (C₈-C₂₀)Alkylbetainen, (C₈-C₂₀)Alkylamido(C₁-C₈)alkylbetainen, (C₈-C₂₀)Alkylamido(C₁-C₈)alkylsulfobetainen, Sulfobetainen oder Mischungen davon ausgewählt sind.

5. Verwendung nach einem der Ansprüche 1 bis 3, **dadurch gekennzeichnet, dass** die Imidazoliumderivate aus Amphocarboxyglycinaten, Amphocarboxypropionaten oder Mischungen davon ausgewählt sind.

6. Verwendung nach einem der Ansprüche 1 bis 3, **dadurch gekennzeichnet, dass** es sich bei den nichtionischen Alkylpyrrolidonen um (C₁-C₃₀)Alkylpyrrolidone handelt.

7. Verwendung nach einem der Ansprüche 1 bis 3, **dadurch gekennzeichnet, dass** die oxyalkylenierten oder glycerinierten Fettalkoholether aus ethoxylierten und/oder propoxylierten oder glycerinierten, gesättigten oder ungesättigten, linearen oder verzweigten, gegebenenfalls hydroxylierten Fettalkoholen mit einer Kette mit 8 bis 30 Kohlenstoffatomen ausgewählt sind.

8. Verwendung nach einem der Ansprüche 1 bis 3, **dadurch gekennzeichnet, dass** die Fettsäureester von oxyalkylenierten oder glycerinierten Monoalkoholen aus Estern von gesättigten oder ungesättigten, linearen oder verzweigten Carbonsäuren mit einer Fettkette mit 8 bis 30 Kohlenstoffatomen und einem oder mehreren ethoxylierten und/oder propoxylierten oder glycerinierten, gesättigten oder ungesättigten, linearen oder verzweigten Monoalkoholen mit einer Fettkette mit 8 bis 30 Kohlenstoffatomen ausgewählt sind.

9. Verwendung nach einem der vorhergehenden Ansprüche, **dadurch gekennzeichnet, dass** das oder die amphoteren Tenside und/oder das oder die nichtionischen Tenside 0,01 bis 30 Gew.-%, spezieller 0,1 bis 20 Gew.-% und vorzugsweise 0,2 bis 10 Gew.-% des Gesamtgewichts der Zusammensetzung ausmachen.

10. Verwendung nach einem der vorhergehenden Ansprüche, **dadurch gekennzeichnet, dass** der gegebenenfalls neutralisierte Fluoreszenzfarbstoff in dem kosmetisch unbedenklichen Medium bei einer Temperatur zwischen 15 und 25°C in einer Menge von mindestens 0,001 g/l, spezieller mindestens 0,5 g/l, vorzugsweise mindestens 1 g/l und gemäß einer noch weiter bevorzugten Ausführungsform mindestens 5 g/l löslich ist.

11. Verwendung nach einem der vorhergehenden Ansprüche, **dadurch gekennzeichnet, dass** der Fluoreszenzfarbstoff zu einem Remissionsmaximum führt, das im Wellenlängenbereich von 500 bis 650 Nanometer und vorzugsweise im Wellenlängenbereich von 550 bis 620 Nanometer liegt.

12. Verwendung nach einem der vorhergehenden Ansprüche, **dadurch gekennzeichnet, dass** der Fluoreszenzfarbstoff aus der Gruppe bestehend aus den Farbstoffen der folgenden Strukturen ausgewählt ist: 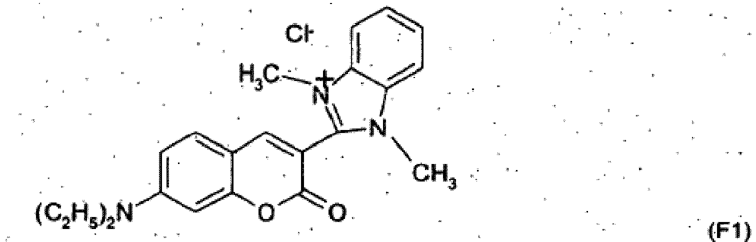 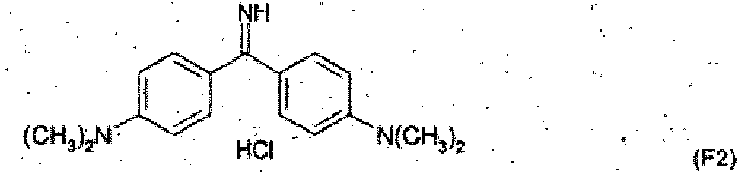 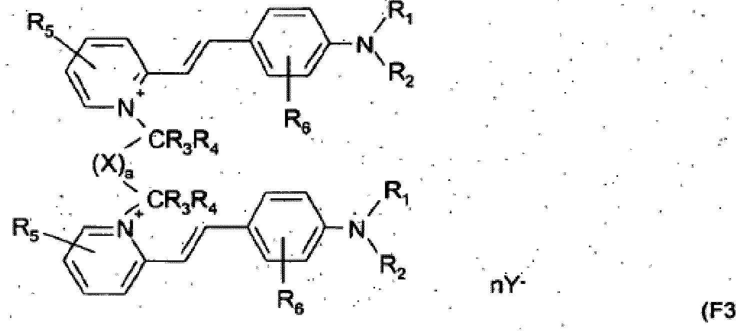 worin:
R₁ und R₂ gleich oder verschieden sind und für:
• ein Wasserstoffatom;
• einen linearen oder verzweigten Alkylrest mit 1 bis 10 Kohlenstoffatomen, vorzugsweise 1 bis 4 Kohlenstoffatomen, der gegebenenfalls durch mindestens ein Heteroatom und/oder mindestens eine Gruppe mit mindestens einem Heteroatom unterbrochen und/oder substituiert ist und/oder durch mindestens ein Halogenatom substituiert ist;
• einen Aryl- oder Arylalkylrest, wobei die Arylgruppe 6 Kohlenstoffatome aufweist und der Alkylrest 1 bis 4 Kohlenstoffatome aufweist; wobei der Arylrest gegebenenfalls durch einen oder mehrere lineare oder verzweigte Alkylreste mit 1 bis 4 Kohlenstoffatomen, die gegebenenfalls durch mindestens ein Heteroatom und/oder mindestens eine Gruppe mit mindestens einem Heteroatom unterbrochen und/oder substituiert sind, und/oder durch mindestens ein Halogenatom substituiert ist;
stehen;
• R₁ und R₂ gegebenenfalls zu einem Heterocyclus mit dem Stickstoff, der ein oder mehrere andere Heteroatome enthalten kann, verbunden sind, wobei der Heterocyclus gegebenenfalls durch mindestens einen linearen oder verzweigten Alkylrest mit 1 bis 4 Kohlenstoffatomen, der gegebenenfalls durch mindestens ein Heteroatom und/oder mindestens eine Gruppe mit mindestens einem Heteroatom unterbrochen und/oder substituiert ist, und/oder durch mindestens ein Halogenatom substituiert ist;
• R₁ oder R₂ gegebenenfalls an einem Heterocyclus beteiligt sein kann, der das Stickstoffatom und eines der Kohlenstoffatome der das Stickstoffatom tragenden Phenylgruppe umfasst;
R₃ und R₄ gleich oder verschieden sind und für ein Wasserstoffatom oder einen Alkylrest mit 1 bis 4 Kohlenstoffatomen stehen;
die Reste R₅ gleich oder verschieden sind und für ein Wasserstoffatom, ein Halogenatom oder einen linearen oder verzweigten Alkylrest mit 1 bis 4 Kohlenstoffatomen, der gegebenenfalls durch mindestens ein Heteroatom unterbrochen ist, stehen;
die Reste R₆ gleich oder verschieden sind und für ein Wasserstoffatom; ein Halogenatom oder einen linearen oder verzweigten Alkylrest mit 1 bis 4 Kohlenstoffatomen, der gegebenenfalls durch mindestens ein Heteroatom und/oder mindestens eine Gruppe mit mindestens einem Heteroatom unterbrochen und/oder substituiert ist und/oder durch mindestens ein Halogenatom substituiert ist, stehen;
X für:
• einen linearen oder verzweigten Alkylrest mit 1 bis 14 Kohlenstoffatomen oder Alkenylrest mit 2 bis 14 Kohlenstoffatomen, der gegebenenfalls durch mindestens ein Heteroatom und/oder mindestens eine Gruppe mit mindestens einem Heteroatom unterbrochen und/oder substituiert ist und/oder durch mindestens ein Halogenatom substituiert ist;
• einen 5- oder 6-gliedrigen heterocyclischen Rest, der gegebenenfalls durch mindestens einen linearen oder verzweigten Alkylrest mit 1 bis 14 Kohlenstoffatomen, der gegebenenfalls durch mindestens ein Heteroatom substituiert ist; durch mindestens einen linearen oder verzweigten Alkylaminorest mit 1 bis 4 Kohlenstoffatomen, der gegebenenfalls durch mindestens ein Heteroatom substituiert ist; durch mindestens ein Halogenatom substituiert ist;
• einen gegebenenfalls anellierten aromatischen oder diaromatischen Rest, der gegebenenfalls durch einen Alkylrest mit 1 bis 4 Kohlenstoffatomen abgetrennt ist, wobei der Arylrest oder die Arylreste gegebenenfalls durch mindestens ein Halogenatom oder durch mindestens einen Alkylrest mit 1 bis 10 Kohlenstoffatomen, der gegebenenfalls durch mindestens ein Heteroatom und/oder mindestens eine Gruppe mit mindestens einem Heteroatom substituiert und/oder unterbrochen ist, substituiert sind;
• einen Dicarbonylrest
steht;
• wobei die Gruppe X eine oder mehrere kationische Ladungen tragen kann;
a gleich 0 oder 1 ist;
die Reste Y⁻ gleich oder verschieden sind und für ein organisches oder anorganisches Anion stehen;
wobei n für eine ganze Zahl mit einem Wert von mindestens 2 und höchstens gleich der Zahl der in der Fluoreszenzverbindung vorliegenden kationischen Ladungen steht; 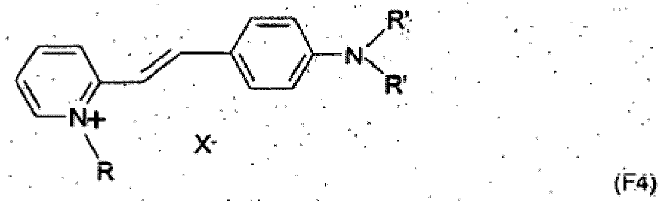 worin R für einen Methyl- oder Ethylrest steht; R' für einen Methylrest steht und X- für ein Anion vom Chlorid-, Iodid-, Sulfat-, Methosulfat-, Acetat- oder Perchlorat-Typ steht.

13. Verwendung nach einem der vorhergehenden Ansprüche, **dadurch gekennzeichnet, dass** der bzw. die Fluoreszenzfarbstoffe in einer Gewichtskonzentration von 0,01 bis 20 Gew.-%, spezieller 0,05 bis 10 Gew.-% und vorzugsweise 0,1 bis 5 Gew.-%, bezogen auf das Gesamtgewicht der Zusammensetzung, vorliegen.

14. Verwendung nach einem der vorhergehenden Ansprüche, **dadurch gekennzeichnet, dass** die Zusammensetzung ferner mindestens einen zusätzlichen nichtfluoreszierenden Direktfarbstoff nichtionischer, kationischer oder anionischer Natur umfasst.

15. Verwendung nach Anspruch 14, **dadurch gekennzeichnet, dass** die zusätzlichen Direktfarbstoffe aus Nitrobenzol-Farbstoffen, Azo-Farbstoffen, Anthrachinon-, Naphthochinon- oder Benzochinon-Farbstoffen, Indigoid-Farbstoffen oder von Triarylmethan abgeleiteten Farbstoffen oder Mischungen davon ausgewählt sind.

16. Verwendung nach einem der Ansprüche 14 oder 15, **dadurch gekennzeichnet, dass** die zusätzlichen Direktfarbstoffe 0,0005 bis 12 Gew.-% und vorzugsweise 0,005 bis 6 Gew.-%, bezogen auf das Gesamtgewicht der Zusammensetzung, ausmachen.

17. Verwendung nach einem der vorhergehenden Ansprüche, **dadurch gekennzeichnet, dass** sie in Form eines aufhellenden Färbeshampoos vorliegt.

18. Verwendung nach einem der vorhergehenden Ansprüche, **dadurch gekennzeichnet, dass** die folgenden Schritte durchgeführt werden:
a) die Zusammensetzung, die mindestens einen Fluoreszenzfarbstoff und mindestens ein amphoteres und/oder nichtionisches Tensid umfasst, wird über einen zur Entwicklung der gewünschten Färbung und Aufhellung ausreichenden Zeitraum auf die Keratinfasern aufgebracht,
b) die Fasern werden gegebenenfalls gespült,
c) die Fasern werden gegebenenfalls mit Shampoo gewaschen und gespült,
d) die Fasern werden getrocknet oder trocknen gelassen.

19. Verwendung nach einem der Ansprüche 1 bis 15, **dadurch gekennzeichnet, dass** man die Zusammensetzung, die mindestens einen Fluoreszenzfarbstoff und mindestens ein amphoteres und/oder nichtionisches Tensid umfasst, auf die Haut aufbringt und die Haut dann trocknet oder trocknen lässt.

20. Zusammensetzung, **dadurch gekennzeichnet, dass** sie in einem kosmetisch unbedenklichen Medium mindestens einen in dem Medium löslichen Fluoreszenzfarbstoff umfasst, der aus der Gruppe bestehend aus den Farbstoffen der folgenden Strukturen ausgewählt ist: 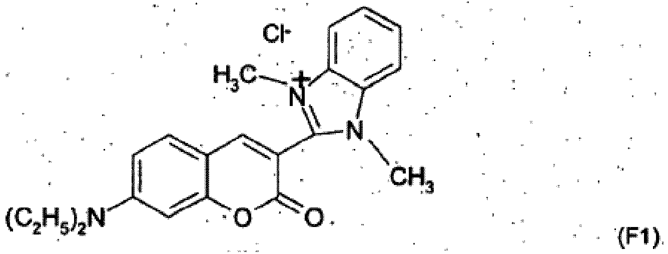 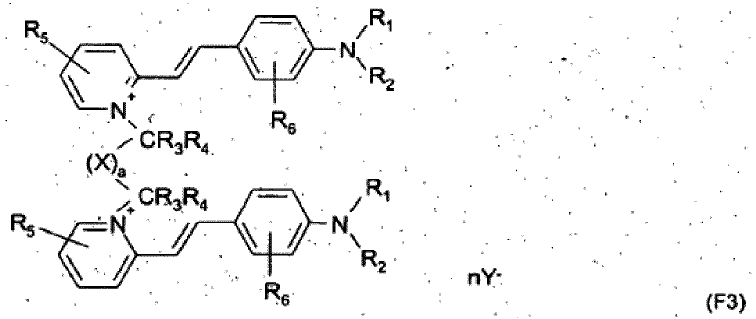 worin:
R₁ und R₂ gleich oder verschieden sind und für:
• ein Wasserstoffatom;
• einen linearen oder verzweigten Alkylrest mit 1 bis 10 Kohlenstoffatomen, vorzugsweise 1 bis 4 Kohlenstoffatomen, der gegebenenfalls durch mindestens ein Heteroatom und/oder mindestens eine Gruppe mit mindestens einem Heteroatom unterbrochen und/oder substituiert ist und/oder durch mindestens ein Halogenatom substituiert ist;
• einen Aryl- oder Arylalkylrest, wobei die Arylgruppe 6 Kohlenstoffatome aufweist und der Alkylrest 1 bis 4 Kohlenstoffatome aufweist; wobei der Arylrest gegebenenfalls durch einen oder mehrere lineare oder verzweigte Alkylreste mit 1 bis 4 Kohlenstoffatomen, die gegebenenfalls durch mindestens ein Heteroatom und/oder mindestens eine Gruppe mit mindestens einem Heteroatom unterbrochen und/oder substituiert sind, und/oder durch mindestens ein Halogenatom substituiert ist;
stehen;
• R₁ und R₂ gegebenenfalls zu einem Heterocyclus mit dem Stickstoff, der ein oder mehrere andere Heteroatome enthalten kann, verbunden sind, wobei der Heterocyclus gegebenenfalls durch mindestens einen linearen oder verzweigten Alkylrest mit 1 bis 4 Kohlenstoffatomen, der gegebenenfalls durch mindestens ein Heteroatom und/oder mindestens eine Gruppe mit mindestens einem Heteroatom unterbrochen und/oder substituiert ist, und/oder durch mindestens ein Halogenatom substituiert ist;
• R₁ oder R₂ gegebenenfalls an einem Heterocyclus beteiligt sein kann, der das Stickstoffatom und eines der Kohlenstoffatome der das Stickstoffatom tragenden Phenylgruppe umfasst;
R₃ und R₄ gleich oder verschieden sind und für ein Wasserstoffatom oder einen Alkylrest mit 1 bis 4 Kohlenstoffatomen stehen;
die Reste R₅ gleich oder verschieden sind und für ein Wasserstoffatom, ein Halogenatom oder einen linearen oder verzweigten Alkylrest mit 1 bis 4 Kohlenstoffatomen, der gegebenenfalls durch mindestens ein Heteroatom unterbrochen ist, stehen;
die Reste R₆ gleich oder verschieden sind und für ein Wasserstoffatom; ein Halogenatom oder einen linearen oder verzweigten Alkylrest mit 1 bis 4 Kohlenstoffatomen, der gegebenenfalls durch mindestens ein Heteroatom und/oder mindestens eine Gruppe mit mindestens einem Heteroatom unterbrochen und/oder substituiert ist und/oder durch mindestens ein Halogenatom substituiert ist, stehen;
X für:
• einen linearen oder verzweigten Alkylrest mit 1 bis 14 Kohlenstoffatomen oder Alkenylrest mit 2 bis 14 Kohlenstoffatomen, der gegebenenfalls durch mindestens ein Heteroatom und/oder mindestens eine Gruppe mit mindestens einem Heteroatom unterbrochen und/oder substituiert ist und/oder durch mindestens ein Halogenatom substituiert ist;
• einen 5- oder 6-gliedrigen heterocyclischen Rest, der gegebenenfalls durch mindestens einen linearen oder verzweigten Alkylrest mit 1 bis 14 Kohlenstoffatomen, der gegebenenfalls durch mindestens ein Heteroatom substituiert ist; durch mindestens einen linearen oder verzweigten Alkylaminorest mit 1 bis 4 Kohlenstoffatomen, der gegebenenfalls durch mindestens ein Heteroatom substituiert ist; durch mindestens ein Halogenatom substituiert ist;
• einen gegebenenfalls anellierten aromatischen oder diaromatischen Rest, der gegebenenfalls durch einen Alkylrest mit 1 bis 4 Kohlenstoffatomen abgetrennt ist, wobei der Arylrest oder die Arylreste gegebenenfalls durch mindestens ein Halogenatom oder durch mindestens einen Alkylrest mit 1 bis 10 Kohlenstoffatomen, der gegebenenfalls durch mindestens ein Heteroatom und/oder mindestens eine Gruppe mit mindestens einem Heteroatom substituiert und/oder unterbrochen ist, substituiert sind;
• einen Dicarbonylrest
steht;
• wobei die Gruppe X eine oder mehrere kationische Ladungen tragen kann;
a gleich 0 oder 1 ist;
die Reste Y⁻ gleich oder verschieden sind und für ein organisches oder anorganisches Anion stehen;
wobei n für eine ganze Zahl mit einem Wert von mindestens 2 und höchstens gleich der Zahl der in der Fluoreszenzverbindung vorliegenden kationischen Ladungen steht; wobei die Zusammensetzung nicht 2-[2-(4-Dialkylamino)phenylethenyl]-1-alkyl-pyridinium, worin der Alkylrest des Pyridiniumkerns für einen Methyl- oder Ethylrest steht, der Alkylrest des Benzolkerns für einen Methylrest steht und es sich bei dem Gegenion um ein Halogenid handelt, als fluoreszierendes Mittel umfasst; und
mindestens ein amphoteres Tensid, ausgewählt aus Betainen und Imidazoliumderivaten, und/oder mindestens ein nichtionisches Tensid, ausgewählt aus Alkylpyrrolidonen, oxyalkylenierten oder glycerinierten Fettalkoholethern, Fettsäureestern von oxyalkylenierten oder glycerinierten Monoalkoholen und Fettsäureestern und Polyolen, in Abwesenheit von Oxidationsfarbstoffen und Oxidationsmitteln umfasst.

21. Zusammensetzung nach dem vorhergehenden Anspruch, **dadurch gekennzeichnet, dass** der gegebenenfalls neutralisierte Fluoreszenzfarbstoff in dem Medium der Zusammensetzung bei einer Temperatur zwischen 15 und 25°C in einer Menge von mindestens 0,001 g/l, spezieller mindestens 0,5 g/l, vorzugsweise mindestens 1 g/l und gemäß einer noch weiter bevorzugten Ausführungsform mindestens 5 g/l löslich ist.

22. Zusammensetzung nach Anspruch 20, **dadurch gekennzeichnet, dass** es sich bei dem Fluoreszenzfarbstoff um einen Farbstoff im Orangebereich handelt.

23. Zusammensetzung nach einem der Ansprüche 20 bis 21, **dadurch gekennzeichnet, dass** der Fluoreszenzfarbstoff zu einem Remissionsmaximum führt, das im Wellenlängenbereich von 500 bis 650 Nanometer und vorzugsweise im Wellenlängenbereich von 550 bis 620 Nanometer liegt.

24. Zusammensetzung nach einem der Ansprüche 20 bis 23, **dadurch gekennzeichnet, dass** die amphoteren Tenside vom Betain-Typ aus (C₈-C₂₀) Alkylbetainen, (C₈-C₂₀)Alkylamido (C₁-C₈)alkylbetainen, (C₈-C₂₀)Alkylamido(C₁-C₈) alkylsulfobetainen, Sulfobetainen oder Mischungen davon ausgewählt sind.

25. Zusammensetzung nach einem der Ansprüche 20 bis 23, **dadurch gekennzeichnet, dass** die Imidazoliumderivate aus Amphocarboxyglycinaten, Amphocarboxypropionaten oder Mischungen davon ausgewählt sind.

26. Zusammensetzung nach einem der Ansprüche 20 bis 24, **dadurch gekennzeichnet, dass** es sich bei den nichtionischen Alkylpyrrolidonen um (C₁-C₃₀)Alkyl-pyrrolidone handelt.

27. Zusammensetzung nach einem der Ansprüche 20 bis 25, **dadurch gekennzeichnet, dass** die oxyalkylenierten oder glycerinierten Fettalkoholether aus ethoxylierten und/oder propoxylierten oder glycerinierten, gesättigten oder ungesättigten, linearen oder verzweigten, gegebenenfalls hydroxylierten Fettalkoholen mit einer Kette mit 8 bis 30 Kohlenstoffatomen ausgewählt sind.

28. Zusammensetzung nach einem der Ansprüche 20 bis 26, **dadurch gekennzeichnet, dass** die Fettsäureester von oxyalkylenierten oder glycerinierten Monoalkoholen aus Estern von gesättigten oder ungesättigten, linearen oder verzweigten Carbonsäuren mit einer Kette mit 8 bis 30 Kohlenstoffatomen und einem ethoxylierten und/oder propoxylierten oder glycerinierten, gesättigten oder ungesättigten, linearen oder verzweigten Monoalkohol mit einer Kette mit 8 bis 30 Kohlenstoffatomen ausgewählt sind.

29. Zusammensetzung nach einem der Ansprüche 20 bis 27, **dadurch gekennzeichnet, dass** das bzw. die Polyole aus Glycerin, Sorbitol, Glucose, Methylglucose, Sorbitolanhydrid, einem Polyethylenglykol oder einem Polypropylenglykol oder Mischungen davon ausgewählt sind.

30. Zusammensetzung nach einem der Ansprüche 20 bis 28, **dadurch gekennzeichnet, dass** das oder die amphoteren Tenside und/oder die nichtionischen Tenside 0,01 bis 30 Gew.-%, spezieller 0,1 bis 20 Gew.-% und vorzugsweise 0,2 bis 10 Gew.-% des Gesamtgewichts der Zusammensetzung ausmachen.

31. Zusammensetzung nach einem der Ansprüche 20 bis 29, **dadurch gekennzeichnet, dass** der bzw. die Fluoreszenzfarbstoffe in einer Gewichtskonzentration von 0,01 bis 20 Gew.-%, spezieller 0,05 bis 10 Gew.-% und vorzugsweise 0,1 bis 5 Gew.-%, bezogen auf das Gesamtgewicht der Zusammensetzung, vorliegen.

32. Zusammensetzung nach einem der Ansprüche 20 bis 30, **dadurch gekennzeichnet, dass** sie ferner mindestens einen zusätzlichen nichtfluoreszierenden Direktfarbstoff nichtionischer, kationischer oder anionischer Natur umfasst.

33. Zusammensetzung nach Anspruch 31, **dadurch gekennzeichnet, dass** die zusätzlichen Direktfarbstoffe aus Nitrobenzol-Farbstoffen, Azo-Farbstoffen, Anthrachinon-Farbstoffen, Naphthochinon-Farbstoffen, Benzochinon-Farbstoffen, Phenotiazin-Farbstoffen, Indigoid-Farbstoffen, Xanthen-Farbstoffen, Phenanthridin-Farbstoffen, Phthalocyanin-Farbstoffen sowie von Triarylmethan abgeleiteten Farbstoffen allein oder Mischungen davon ausgewählt sind.

34. Zusammensetzung nach einem der Ansprüche 31 oder 32, **dadurch gekennzeichnet, dass** der bzw. die zusätzlichen Direktfarbstoffe 0,0005 bis 12 Gew.-% und vorzugsweise 0,005 bis 6 Gew.-%, bezogen auf das Gesamtgewicht der Zusammensetzung, ausmachen.

35. Zusammensetzung nach einem der Ansprüche 20 bis 33, **dadurch gekennzeichnet, dass** sie in Form eines aufhellenden Färbeshampoos vorliegt.

36. Verfahren zum aufhellend wirkenden Färben von pigmentierten oder künstlich gefärbten menschlichen Keratinfasern, **dadurch gekennzeichnet, dass** die folgenden Schritte durchgeführt werden:
a) eine Zusammensetzung nach einem der Ansprüche 20 bis 33 und 35 wird über einen zur Entwicklung der gewünschten Färbung und Aufhellung ausreichenden Zeitraum auf die Fasern aufgebracht,
b) die Fasern werden gegebenenfalls gespült,
c) die Fasern werden gegebenenfalls mit Shampoo gewaschen und gespült,
d) die Fasern werden getrocknet oder trocknen gelassen,
in Abwesenheit von Oxidationsfarbstoffen und Oxidationsmitteln.

## Claims

1. Use of a composition comprising, in a cosmetically acceptable medium, at least one dye that is fluorescent in the orange range, which is soluble in the medium, chosen from the fluorescent dyes belonging to the following families: naphthalimides; cationic or non-cationic coumarins; xanthenodiquinolizines; azaxanthenes; naphtholactams; azlactones; oxazines; thiazines; dioxazines; monocationic or polycationic fluorescent dyes of azo, azomethine or methine type, and at least one amphoteric surfactant chosen from betaines and imidazolium derivatives and/or at least one nonionic surfactant chosen from alkylpyrrolidones, oxyalkylenated or glycerolated fatty alcohol ethers, and oxyalkylenated or glycerolated fatty acid esters of monoalcohols, for the dyeing with a lightening effect of artificially coloured or pigmented human keratin fibres, in the absence of oxidation dyes and of oxidizing agents.

2. Use according to either of Claims 1 and 2, **characterized in that** said fibres have a tone depth of less than or equal to 6 and preferably less than or equal to 4.

3. Use according to one of Claims 1 to 3, for dyeing hair with a lightening effect, with a lightening effect of at least 0.5 tone.

4. Use according to any one of the preceding claims, **characterized in that** the amphoteric surfactants of betaine type are chosen from (C₈-C₂₀) alkylbetaines, (C₈-C₂₀) alkylamido (C₁-C₈)alkylbetaines, (C₈-C₂₀) alkylamido (C₁-C₈)alkylsulfobetaines and sulfobetaines, or mixtures thereof.

5. Use according to any one of Claims 1 to 3, **characterized in that** the imidazolium derivatives are chosen from amphocarboxyglycinates and amphocarboxypropionates, or mixtures thereof.

6. Use according to any one of Claims 1 to 3, **characterized in that** the nonionic alkylpyrrolidones are (C₁-C₃₀)alkylpyrrolidones.

7. Use according to any one of Claims 1 to 3, **characterized in that** the oxyalkylenated or glycerolated fatty alcohol ethers are chosen from linear or branched, saturated or unsaturated, ethoxylated and/or propoxylated or glycerolated, optionally hydroxylated fatty alcohols, with a chain comprising from 8 to 30 carbon atoms.

8. Use according to any one of Claims 1 to 3, **characterized in that** the oxyalkylenated or glycerolated fatty acid esters of monoalcohols are chosen from esters of linear or branched, saturated or unsaturated carboxylic acids with a fatty chain comprising from 8 to 30 carbon atoms and of one or more linear or branched, saturated or unsaturated, ethoxylated and/or propoxylated or glycerolated monoalcohols with a fatty chain comprising from 8 to 30 carbon atoms.

9. Use according to any one of the preceding claims, **characterized in that** the amphoteric surfactant(s) and/or the nonionic surfactant(s) represent from 0.01% to 30% by weight, more particularly from 0.1% to 20% by weight and preferably from 0.2% to 10% by weight relative to the total weight of the composition.

10. Use according to any one of the preceding claims, **characterized in that** the optionally neutralized fluorescent dye is soluble in the cosmetically acceptable medium to at least 0.001 g/l, more particularly at least 0.5 g/l, preferably at least 1 g/l, and according to an even more preferred embodiment, at least 5 g/l at a temperature of between 15 and 25°C.

11. Use according to any one of the preceding claims, **characterized in that** the fluorescent dye leads to a reflectance maximum that is in the wavelength range from 500 to 650 nanometres and preferably in the wavelength range from 550 to 620 nanometres.

12. Use according to any one of the preceding claims, **characterized in that** the fluorescent dye is chosen from the group formed by the dyes having the following structures: 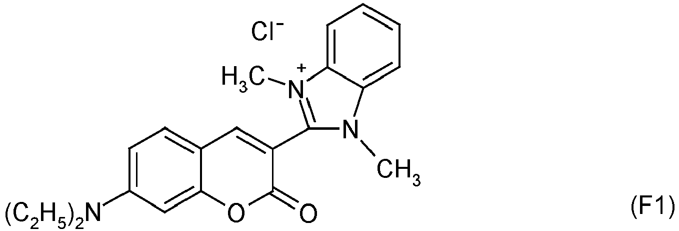 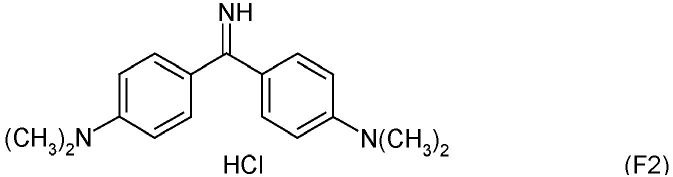 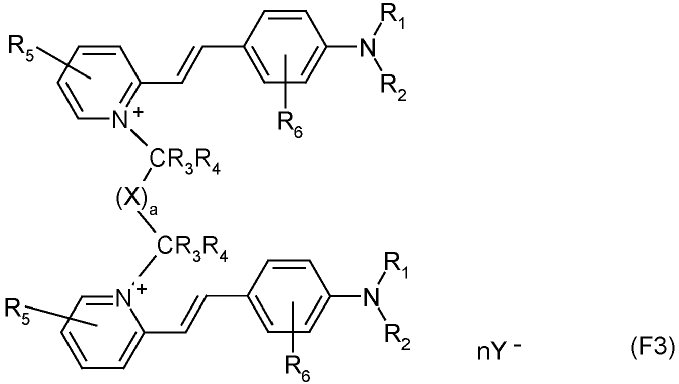 in which:
R₁ and R₂, which may be identical or different, represent:
• a hydrogen atom;
• a linear or branched alkyl radical comprising 1 to 10 carbon atoms and preferably from 1 to 4 carbon atoms, optionally interrupted and/or substituted with at least one heteroatom and/or group comprising at least one heteroatom and/or substituted with at least one halogen atom;
• an aryl or arylalkyl radical, the aryl group containing 6 carbon atoms and the alkyl radical containing 1 to 4 carbon atoms; the aryl radical optionally being substituted with one or more linear or branched alkyl radicals comprising 1 to 4 carbon atoms optionally interrupted and/or substituted with at least one heteroatom and/or group comprising at least one heteroatom and/or substituted with at least one halogen atom;
• R₁ and R₂ may optionally be linked so as to form a heterocycle with the nitrogen atom and may comprise one or more other heteroatoms, the heterocycle optionally being substituted with at least one linear or branched alkyl radical preferably comprising from 1 to 4 carbon atoms and optionally being interrupted and/or substituted with at least one heteroatom and/or group comprising at least one heteroatom and/or substituted with at least one halogen atom;
• R₁ or R₂ may optionally be engaged in a heterocycle comprising the nitrogen atom and one of the carbon atoms of the phenyl group bearing said nitrogen atom;
R₃ and R₄, which may be identical or different, represent a hydrogen atom or an alkyl radical comprising 1 to 4 carbon atoms;
R₅, which may be identical or different, represent a hydrogen atom, a halogen atom or a linear or branched alkyl radical comprising 1 to 4 carbon atoms, optionally interrupted with at least one heteroatom;
R₆, which may be identical or different, represent a hydrogen atom; a halogen atom; a linear or branched alkyl radical comprising 1 to 4 carbon atoms, optionally substituted and/or interrupted with at least one heteroatom and/or group bearing at least one heteroatom and/or substituted with at least one halogen atom;
X represents:
• a linear or branched alkyl radical comprising 1 to 14 carbon atoms or an alkenyl radical comprising 2 to 14 carbon atoms, optionally interrupted and/or substituted with at least one heteroatom and/or group comprising at least one heteroatom and/or substituted with at least one halogen atom;
• a 5- or 6-membered heterocyclic radical optionally substituted with at least one linear or branched alkyl radical comprising 1 to 14 carbon atoms, optionally substituted with at least one heteroatom; with at least one linear or branched aminoalkyl radical comprising 1 to 4 carbon atoms, optionally substituted with at least one heteroatom; with at least one halogen atom;
• a fused or non-fused aromatic or diaromatic radical, optionally separated by an alkyl radical comprising 1 to 4 carbon atoms, the aryl radical(s) optionally being substituted with at least one halogen atom or with at least one alkyl radical comprising 1 to 10 carbon atoms optionally substituted and/or interrupted with at least one heteroatom and/or group bearing at least one heteroatom;
• a dicarbonyl radical;
• the group X possibly bearing one or more cationic charges;
a being equal to 0 or 1;
Y⁻, which may be identical or different, representing an organic or mineral anion;
n being an integer at least equal to 2 and at most equal to the number of cationic charges present in the fluorescent compound; 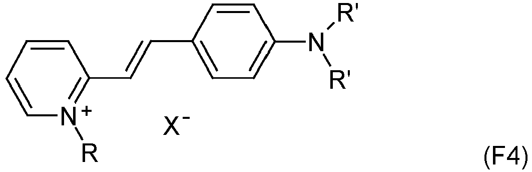 in which formula R represents a methyl or ethyl radical;
R' represents a methyl radical, X⁻ an anion such as chloride, iodide, sulfate, methosulfate, acetate or perchlorate.

13. Use according to any one of the preceding claims, **characterized in that** the fluorescent dye(s) are present in a weight concentration ranging from 0.01% to 20% by weight, more particularly from 0.05% to 10% by weight and preferably from 0.1% to 5% by weight relative to the total weight of the composition.

14. Use according to any one of the preceding claims, **characterized in that** the composition also comprises at least one additional non-fluorescent direct dye of nonionic, cationic or anionic nature.

15. Use according to Claim 14, **characterized in that** the additional direct dyes are chosen from nitrobenzene dyes, azo dyes, anthraquinone dyes, naphthoquinone dyes, benzoquinone dyes, indigoid dyes and triarylmethane-based dyes, or mixtures thereof.

16. Use according to either of Claims 14 and 15, **characterized in that** the additional direct dye(s) represent from 0.0005% to 12% by weight and preferably from 0.005% to 6% by weight relative to the total weight of the composition.

17. Use according to any one of the preceding claims, **characterized in that** the composition is in the form of a lightening and colouring shampoo.

18. Use according to any one of the preceding claims, **characterized in that** the following steps are performed:
a) the composition comprising at least one fluorescent dye and at least one amphoteric and/or nonionic surfactant is applied to keratin fibres, for a time that is sufficient to develop the desired colouring and lightening,
b) said fibres are optionally rinsed,
c) said fibres are optionally washed with shampoo and rinsed,
d) the fibres are dried or left to dry.

19. Use according to any one of Claims 1 to 15, **characterized in that** the composition comprising at least one fluorescent dye and at least one amphoteric and/or nonionic surfactant is applied to the skin, and the skin is then dried or left to dry.

20. Composition **characterized in that** it comprises, in a cosmetically acceptable medium, at least one fluorescent dye that is soluble in the medium, chosen from the group formed by the dyes having the following structures: 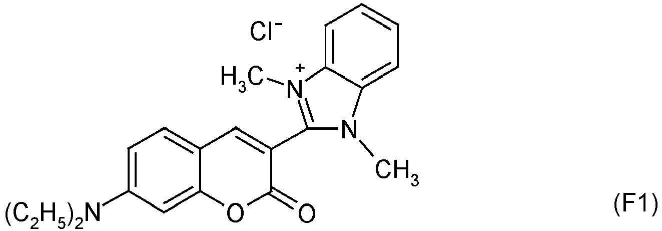 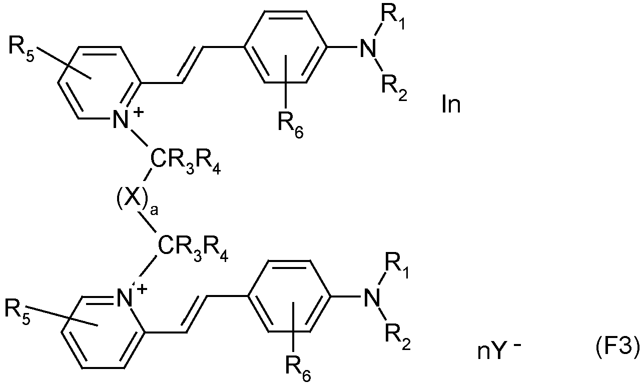 in which:
R₁ and R₂, which may be identical or different, represent:
• a hydrogen atom;
• a linear or branched alkyl radical comprising 1 to 10 carbon atoms and preferably from 1 to 4 carbon atoms, optionally interrupted and/or substituted with at least one heteroatom and/or group comprising at least one heteroatom and/or substituted with at least one halogen atom;
• an aryl or arylalkyl radical, the aryl group containing 6 carbon atoms and the alkyl radical containing 1 to 4 carbon atoms; the aryl radical optionally being substituted with one or more linear or branched alkyl radicals comprising 1 to 4 carbon atoms optionally interrupted and/or substituted with at least one heteroatom and/or group comprising at least one heteroatom and/or substituted with at least one halogen atom;
• R₁ and R₂ may optionally be linked so as to form a heterocycle with the nitrogen atom and may comprise one or more other heteroatoms, the heterocycle optionally being substituted with at least one linear or branched alkyl radical preferably comprising from 1 to 4 carbon atoms and optionally being interrupted and/or substituted with at least one heteroatom and/or group comprising at least one heteroatom and/or substituted with at least one halogen atom;
• R₁ or R₂ may optionally be engaged in a heterocycle comprising the nitrogen atom and one of the carbon atoms of the phenyl group bearing said nitrogen atom;
R₃ and R₄, which may be identical or different, represent a hydrogen atom or an alkyl radical comprising 1 to 4 carbon atoms;
R₅, which may be identical or different, represent a hydrogen atom, a halogen atom or a linear or branched alkyl radical comprising 1 to 4 carbon atoms, optionally interrupted with at least one heteroatom;
R₆, which may be identical or different, represent a hydrogen atom; a halogen atom; a linear or branched alkyl radical comprising 1 to 4 carbon atoms, optionally substituted and/or interrupted with at least one heteroatom and/or group bearing at least one heteroatom and/or substituted with at least one halogen atom;
X represents:
• a linear or branched alkyl radical comprising 1 to 14 carbon atoms or an alkenyl radical comprising 2 to 14 carbon atoms, optionally interrupted and/or substituted with at least one heteroatom and/or group comprising at least one heteroatom and/or substituted with at least one halogen atom;
• a 5- or 6-membered heterocyclic radical optionally substituted with at least one linear or branched alkyl radical comprising 1 to 14 carbon atoms, optionally substituted with at least one heteroatom; with at least one linear or branched aminoalkyl radical comprising 1 to 4 carbon atoms, optionally substituted with at least one heteroatom; with at least one halogen atom;
• a fused or non-fused aromatic or diaromatic radical, optionally separated by an alkyl radical comprising 1 to 4 carbon atoms, the aryl radical(s) optionally being substituted with at least one halogen atom or with at least one alkyl radical comprising 1 to 10 carbon atoms optionally substituted and/or interrupted with at least one heteroatom and/or group bearing at least one heteroatom;
• a dicarbonyl radical;
• the group X possibly bearing one or more cationic charges;
a being equal to 0 or 1;
Y⁻, which may be identical or different, representing an organic or mineral anion;
n being an integer at least equal to 2 and at most equal to the number of cationic charges present in the fluorescent compound, the composition not comprising, as fluorescent agent, 2-[2-(4-dialkylamino)phenylethenyl]-1-alkylpyridinium in which the alkyl radical of the pyridinium nucleus represents a methyl or ethyl radical, that of the benzene nucleus represents a methyl radical and in which the counterion is a halide; and at least one amphoteric surfactant chosen from betaines and imidazolium derivatives and/or at least one nonionic surfactant chosen from alkylpyrrolidones, oxyalkylenated or glycerolated fatty alcohol ethers, oxyalkylenated or glycerolated fatty acid esters of monoalcohols and fatty acid esters of polyols,
in the absence of oxidation dyes and of oxidizing agents.

21. Composition according to the preceding claim, **characterized in that** the optionally neutralized fluorescent dye is soluble in the medium of the composition to at least 0.001 g/l, more particularly at least 0.5 g/l, preferably at least 1 g/l and, according to an even more preferred embodiment, at least 5 g/l at a temperature of between 15 and 25°C.

22. Composition according to Claim 20, **characterized in that** the fluorescent dye is a dye in the orange range.

23. Composition according to either of Claims 20 and 21, **characterized in that** the fluorescent dye leads to a reflectance maximum that is in the wavelength range from 500 to 650 nanometres and preferably in the wavelength range from 550 to 620 nanometres.

24. Composition according to one of Claims 20 to 23, **characterized in that** the amphoteric surfactants of betaine type are chosen from (C₈-C₂₀)alkylbetaines, (C₈-C₂₀)alkylamido (C₁-C₈)alkylbetaines, (C₈-C₂₀)alkylamido (C₁-C₈)alkylsulfobetaines and sulfobetaines, or mixtures thereof.

25. Composition according to one of Claims 20 to 23, **characterized in that** the imidazolium derivatives are chosen from amphocarboxyglycinates and amphocarboxypropionates, or mixtures thereof.

26. Composition according to one of Claims 20 to 24, **characterized in that** the nonionic alkylpyrrolidones are (C₁-C₃₀)alkylpyrrolidones.

27. Composition according to one of Claims 20 to 25, **characterized in that** the oxyalkylenated or glycerolated fatty alcohol ethers are chosen from linear or branched, saturated or unsaturated, ethoxylated and/or propoxylated or glycerolated, optionally hydroxylated fatty alcohols, with a chain comprising from 8 to 30 carbon atoms.

28. Composition according to one of Claims 20 to 26, **characterized in that** the oxyalkylenated or glycerolated fatty acid esters of monoalcohols are chosen from esters of linear or branched, saturated or unsaturated carboxylic acids with a chain comprising from 8 to 30 carbon atoms and of a linear or branched, saturated or unsaturated, ethoxylated and/or propoxylated or glycerolated monoalcohol with a chain comprising from 8 to 30 carbon atoms.

29. Composition according to one of Claims 20 to 27, **characterized in that** the polyol(s) are chosen from glycerol, sorbitol, glucose, methylglucose, sorbitol anhydride, a polyethylene glycol or a polypropylene glycol, or mixtures thereof.

30. Composition according to any one of Claims 20 to 28, **characterized in that** the amphoteric surfactant(s) and/or the nonionic surfactants represent from 0.01% to 30% by weight, more particularly from 0.1% to 20% by weight and preferably from 0.2% to 10% by weight relative to the total weight of the composition.

31. Composition according to any one of Claims 20 to 29, **characterized in that** the fluorescent dye(s) are present in a weight concentration ranging from 0.01% to 20% by weight, more particularly from 0.05% to 10% by weight and preferably from 0.1% to 5% by weight relative to the total weight of the composition.

32. Composition according to any one of Claims 20 to 30, **characterized in that** it comprises at least one additional non-fluorescent direct dye of nonionic, cationic or anionic nature.

33. Composition according to Claim 31, **characterized in that** the additional direct dyes are chosen from nitrobenzene dyes, azo dyes, anthraquinone dyes, naphthoquinone dyes, benzoquinone dyes, phenothiazine dyes, indigoid, xanthene, phenanthridine or phthalocyanine dyes, and also triarylmethane-based dyes, alone or as mixtures.

34. Composition according to either of Claims 31 and 32, **characterized in that** the additional direct dye(s) represent from 0.0005% to 12% by weight and preferably from 0.005% to 6% by weight relative to the total weight of the composition.

35. Composition according to any one of Claims 20 to 33, **characterized in that** it is in the form of a lightening and colouring shampoo.

36. Process for the dyeing with a lightening effect of artificially coloured or pigmented human keratin fibres, **characterized in that** the following steps are performed:
a) a composition as defined according to any one of Claims 20 to 33 and 35 is applied to said fibres, for a time that is sufficient to develop the desired colouring and lightening,
b) the fibres are optionally rinsed,
c) the fibres are optionally washed with shampoo and rinsed,
d) the fibres are dried or left to dry,
in the absence of oxidation dyes and of oxidizing agents.
